# EUROPEAN PATENT SPECIFICATION

(11) **EP 1 892 291 B1**
(45) Date of publication and mention of the grant of the patent: **04.05.2011**
(21) Application number: 06017649.2
(22) Date of filing: 24.08.2006
(51) Int. Cl.: C12N 9/12

(54) **Human ron-related gene variant associated with cancers**
Mit Krebs assoziierte menschliche RON verwandte Genvariante
Variantes du gène humain RON liées au cancer

(43) Date of publication of application: 27.02.2008
(73) Proprietor: Visgeneer, Inc., Hsinchu City 300 (TW)
(72) Inventor: Dai, Ken-Shwo, Hsinchu City 300, TAIWAN (TW)
(74) Representative: Epping - Hermann - Fischer

(56) References cited:
- DATABASE EMBL [Online] embo; 12 April 1999 (1999-04-12), SUNDAY ME, WILLET C;: "Human RON receptor protein." XP002411435 retrieved from EBI accession no. AAW82791 Database accession no. AAW82791
- ZHOU YONG-QING ET AL: "Altered expression of the RON receptor tyrosine kinase in primary human colorectal adenocarcinomas: Generation of different splicing RON variants and their oncogenic potential." ONCOGENE, vol. 22, no. 2, 16 January 2003 (2003-01-16), pages 186-197, XP002411430 ISSN: 0950-9232
- COLLESI CHIARA ET AL: "A splicing variant of the RON transcript induces constitutive tyrosine kinase activity and an invasive phenotype" MOLECULAR AND CELLULAR BIOLOGY, vol. 16, no. 10, 1996, pages 5518-5526, XP002411431 ISSN: 0270-7306
- XU XIANG-MING ET AL: "RNA-mediated gene silencing of the RON receptor tyrosine kinase alters oncogenic phenotypes of human colorectal carcinoma cells." ONCOGENE, vol. 23, no. 52, 4 November 2004 (2004-11-04), pages 8464-8474, XP002411432 ISSN: 0950-9232
- MAGGIORA P ET AL: "OVEREXPRESSION OF THE RON GENE IN HUMAN BREAST CARCINOMA" ONCOGENE, BASINGSTOKE, HANTS, GB, vol. 16, April 1998 (1998-04), pages 2927-2933, XP002911984 ISSN: 0950-9232
- MAZZANTI ROBERTO ET AL: "Differential expression proteomics of human colon cancer" AMERICAN JOURNAL OF PHYSIOLOGY: GASTROINTESTINAL AND LIVER PHYSIOLOGY, AMERICAN PHYSIOLOGICAL SOCIETY, US, vol. 290, no. 6, 1 June 2006 (2006-06-01), pages G1329-G1338, XP009131460 ISSN: 0193-1857 [retrieved on 2006-01-26]

### SEQUENCE LISTING

<110> VISGENEER, INC.
   Dai, Ken-Shwo
<120> HUMAN RON-RELATED GENE VARIANT ASSOCIATED WITH CANCERS
<130> 87189782-002007
<160> 8
<170> PatentIn version 3.3
<210> 1
   <211> 4541
   <212> DNA
   <213> Homo sapiens
<400> 1
   ggatcctcta gggtcccagc tcgcctcgat ggagctcctc ccgccgctgc ctcagtcctt   60
   cctgttgctg ctgctgttgc ctgccaagcc cgcggcgggc gaggactggc agtgcccgcg  120
   caccccctac gcggcctctc gcgactttga cgtgaagtac gtggtgccca gcttctccgc  180
   cggaggcctg gtacaggcca tggtgaccta cgagggcgac agaaatgaga gtgctgtgtt  240
   tgtagccata cgcaatcgcc tgcatgtgct tgggcctgac ctgaagtctg tccagagcct  300
   ggccacgggc cctgctggag accctggctg ccagacgtgt gcagcctgtg gcccaggacc  360
   ccacggccct cccggtgaca cagacacaaa ggtgctggtg ctggatcccg cgctgcctgc  420
   gctggtcagt tgtggctcca gcctgcaggg ccgctgcttc ctgcatgacc tagagcccca  480
   agggacagcc gtgcatctgg cagcgccagc ctgcctcttc tcagcccacc ataaccggcc  540
   cgatgactgc cccgactgtg tggccagccc attgggcacc cgtgtaactg tggttgagca  600
   aggccaggcc tcctatttct acgtggcatc ctcactggac gcagccgtgg ctggcagctt  660
   cagcccacgc tcagtgtcta tcaggcgtct caaggctgac gcctcgggat tcgcaccggg  720
   ctttgtggcg ttgtcagtgc tgcccaagca tcttgtctcc tacagtattg aatacgtgca  780
   cagcttccac acgggagcct tcgtatactt cctgactgta cagccggcca gcgtgacaga  840
   tgatcctagt gccctgcaca cacgcctggc acggcttagc gccactgagc cagagttggg  900
   tgactatcgg gagctggtcc tcgactgcag atttgctcca aaacgcaggc gccggggggc  960
   cccagaaggc ggacagccct accctgtgct gcaggtggcc cactccgctc cagtgggtgc 1020
   ccaacttgcc actgagctga gcatcgccga gggccaggaa gtactatttg gggtctttgt 1080
   gactggcaag gatggtggtc ctggcgtggg ccccaactct gtcgtctgtg ccttccccat 1140
   tgacctgctg gacacactaa ttgatgaggg tgtggagcgc tgttgtgaat ccccagtcca 1200
   tccaggcctc cggcgaggcc tcgacttctt ccagtcgccc agtttttgcc ccaacccgcc 1260
   tggcctggaa gccctcagcc ccaacaccag ctgccgccac ttccctctgc tggtcagtag 1320
   cagcttctca cgtgtggacc tattcaatgg gctgttggga ccagtacagg tcactgcatt 1380
   gtatgtgaca cgccttgaca acgtcacagt ggcacacatg ggcacaatgg atgggcgtat 1440
   cctgcaggtg gagctggtca ggtcactaaa ctacttgctg tatgtgtcca acttctcact 1500
   gggtgacagt gggcagcccg tgcagcggga tgtcagtcgt cttggggacc acctactctt 1560
   tgcctctggg gaccaggttt tccaggtacc tatccgaggc cctggctgcc gccacttcct 1620
   gacctgtggg cgttgcctaa gggcatggca tttcatgggc tgtggctggt gtgggaacat 1680
   gtgcggccag cagaaggagt gtcctggctc ctggcaacag gaccactgcc cacctaagct 1740
   tactgagttc cacccccaca gtggacctct aaggggcagt acaaggctga ccctgtgtgg 1800
   ctccaacttc taccttcacc cttctggtct ggtgcctgag ggaacccatc aggtcactgt 1860
   gggccaaagt ccctgccggc cactgcccaa ggacagctca aaactcagac cagtgccccg 1920
   gaaagacttt gtagaggagt ttgagtgtga actggagccc ttgggcaccc aggcagtggg 1980
   gcctaccaac gtcagcctca ccgtgactaa catgccaccg ggcaagcact tccgggtaga 2040
   cggcacctcc gtgctgagag gcttctcttt catggagcca gtgctgatag cagtgcaacc 2100
   cctctttggc ccacgggcag gaggcacctg tctcactctt gaaggccaga gtctgtctgt 2160
   aggcaccagc cgggctgtgc tggtcaatgg gactgagtgt ctgctagcac gggtcagtga 2220
   ggggcagctt ttatgtgcca caccccctgg ggccacggtg gccagtgtcc cccttagcct 2280
   gcaggtgggg ggtgcccagg tacctggttc ctggaccttc cagtacagag aagaccctgt 2340
   cgtgctaagc atcagcccca actgtggcta catcaactcc cacatcacca tctgtggcca 2400
   gcatctaact tcagcatggc acttagtgct gtcattccat gacgggctta gggcagtgga 2460
   aagcaggtgt gagaggcagc ttccagagca gcagctgtgc cgccttcctg aatatgtggt 2520
   ccgagacccc cagggatggg tggcagggaa tctgagtgcc cgaggggatg gagctgctgg 2580
   ctttacactg cctggctttc gcttcctacc cccaccccat ccacccagtg ccaacctagt 2640
   tccactgaag cctgaggagc atgccattaa gtttgagtat attgggctgg gcgctgtggc 2700
   tgactgtgtg ggtatcaacg tgaccgtggg tggtgagagc tgccagcacg agttccgggg 2760
   ggacatggtt gtctgccccc tgcccccatc cctgcagctt ggccaggatg gtgccccatt 2820
   gcaggtctgc gtagatggtg aatgtcatat cctgggtaga gtggtgcggc cagggccaga 2880
   tggggtccca cagagcacgc tccttggtat cctgctgcct ttgctgctgc ttgtggctgc 2940
   actggcgact gcactggtct tcagctactg gtggcggagg aagcagctag ttcttcctcc 3000
   caacctgaat gacctggcat ccctggacca gactgctgga gccacacccc tgcctattct 3060
   gtactcgggc tctgactaca gaagtggcct tgcactccct gccattgatg gtctggattc 3120
   caccacttgt gtccatggag catccttctc cgatagtgaa gatgaatcct gtgtgccact 3180
   gctgcggaaa gagtccatcc agctaaggga cctggactct gcgctcttgg ctgaggtcaa 3240
   ggatgtgctg attccccatg agcgggtggt cacccacagt gaccgagtca ttggcaaagg   3300
   ccactttgga gttgtctacc acggagaata catagaccag gcccagaatc gaatccaatg   3360
   tgccatcaag tcactaagtc gcatcacaga gatgcagcag gtggaggcct tcctgcgaga   3420
   ggggctgctc atgcgtggcc tgaaccaccc gaatgtgctg gctctcattg gtatcatgtt   3480
   gccacctgag ggcctgcccc atgtgctgct gccctatatg tgccacggtg acctgctcca   3540
   gttcatccgc tcacctcagc ggaaccccac cgtgaaggac ctcatcagct ttggcctgca   3600
   ggtagcccgc ggcatggagt acctggcaga gcagaagttt gtgcacaggg acctggctgc   3660
   gcggaactgc atgctggacg agtcattcac agtcaaggtg gctgactttg gtttggcccg   3720
   cgacatcctg gacagggagt actatagtgt tcaacagcat cgccacgctc gcctacctgt   3780
   gaagtggatg gcgctggaga gcctgcagac ctatagattt accaccaagt ctgatgtgtg   3840
   gtcatttggt gtgctgctgt gggaactgct gacacggggt gccccaccat accgccacat   3900
   tgaccctttt gaccttaccc acttcctggc ccagggtcgg cgcctgcccc agcctgagta   3960
   ttgccctgat tctctgtacc aagtgatgca gcaatgctgg gaggcagacc cagcagtgcg   4020
   acccaccttc agagtactag tgggggaggt ggagcagata gtgtctgcac tgcttgggga   4080
   ccattatgtg cagctgccag caacctacat gaacttgggc cccagcacct cgcatgagat   4140
   gaatgtgcgt ccagaacagc cgcagttctc acccatgcca gggaatgtac gccggccccg   4200
   gccactctca gagcctcctc ggcccacttg acttagttct tgggctggac ctgcttagct   4260
   gccttgagct aaccccaagg ctgcctctgg gccatgccag gccagagcag tggccctcca   4320
   ccttgttcct gccctttaac tttcagaggc aataggtaaa tgggcccatt aggtccctca   4380
   ctccacagag tgagccagtg agggcagtcc tgcaacatgt atttatggag tgcctgctgt   4440
   ggaccctgtc ttctgggcac agtggactca gcagtgacca caccaacact gacccttgaa   4500
   ccaataaagg aacaaatgac tattaaagca caaaaaaaaa a                        4541
<210> 2
   <211> 1400
   <212> PRT
   <213> Homo sapiens
<400> 2
   Met Glu Leu Leu Pro Pro Leu Pro Gln Ser Phe Leu Leu Leu Leu Leu
   1               5                   10                  15
   Leu Pro Ala Lys Pro Ala Ala Gly Glu Asp Trp Gln Cys Pro Arg Thr
               20                  25                  30
   Pro Tyr Ala Ala Ser Arg Asp Phe Asp Val Lys Tyr Val Val Pro Ser
           35                  40                  45
   Phe Ser Ala Gly Gly Leu Val Gln Ala Met Val Thr Tyr Glu Gly Asp
       50                  55                  60
   Arg Asn Glu Ser Ala Val Phe Val Ala Ile Arg Asn Arg Leu His Val
   65                  70                  75                  80
   Leu Gly Pro Asp Leu Lys Ser Val Gln Ser Leu Ala Thr Gly Pro Ala
                   85                  90                  95
   Gly Asp Pro Gly Cys Gln Thr Cys Ala Ala Cys Gly Pro Gly Pro His
               100                 105                 110
   Gly Pro Pro Gly Asp Thr Asp Thr Lys Val Leu Val Leu Asp Pro Ala
           115                 120                 125
   Leu Pro Ala Leu Val Ser Cys Gly Ser Ser Leu Gln Gly Arg Cys Phe
       130                 135                 140
   Leu His Asp Leu Glu Pro Gln Gly Thr Ala Val His Leu Ala Ala Pro
   145                 150                 155                 160
   Ala Cys Leu Phe Ser Ala His His Asn Arg Pro Asp Asp Cys Pro Asp
                   165                 170                 175
   Cys Val Ala Ser Pro Leu Gly Thr Arg Val Thr Val Val Glu Gln Gly
               180                 185                 190
   Gln Ala Ser Tyr Phe Tyr Val Ala Ser Ser Leu Asp Ala Ala Val Ala
           195                 200                 205
   Gly Ser Phe Ser Pro Arg Ser Val Ser Ile Arg Arg Leu Lys Ala Asp
       210                 215                 220
   Ala Ser Gly Phe Ala Pro Gly Phe Val Ala Leu Ser Val Leu Pro Lys
   225                 230                 235                 240
   His Leu Val Ser Tyr Ser Ile Glu Tyr Val His Ser Phe His Thr Gly
                   245                 250                 255
   Ala Phe Val Tyr Phe Leu Thr Val Gln Pro Ala Ser Val Thr Asp Asp
               260                 265                 270
   Pro Ser Ala Leu His Thr Arg Leu Ala Arg Leu Ser Ala Thr Glu Pro
           275                 280                 285
   Glu Leu Gly Asp Tyr Arg Glu Leu Val Leu Asp Cys Arg Phe Ala Pro
   290 295 300
   Lys Arg Arg Arg Arg Gly Ala Pro Glu Gly Gly Gln Pro Tyr Pro Val
   305 310 315 320
   Leu Gln Val Ala His Ser Ala Pro Val Gly Ala Gln Leu Ala Thr Glu
   325 330 335
   Leu Ser Ile Ala Glu Gly Gln Glu Val Leu Phe Gly Val Phe Val Thr
   340 345 350
   Gly Lys Asp Gly Gly Pro Gly Val Gly Pro Asn Ser Val Val Cys Ala
   355 360 365
   Phe Pro Ile Asp Leu Leu Asp Thr Leu Ile Asp Glu Gly Val Glu Arg
   370 375 380
   Cys Cys Glu Ser Pro Val His Pro Gly Leu Arg Arg Gly Leu Asp Phe
   385 390 395 400
   Phe Gln Ser Pro Ser Phe Cys Pro Asn Pro Pro Gly Leu Glu Ala Leu
   405 410 415
   Ser Pro Asn Thr Ser Cys Arg His Phe Pro Leu Leu Val Ser Ser Ser
   420 425 430
   Phe Ser Arg Val Asp Leu Phe Asn Gly Leu Leu Gly Pro Val Gln Val
   435 440 445
   Thr Ala Leu Tyr Val Thr Arg Leu Asp Asn Val Thr Val Ala His Met
   450 455 460
   Gly Thr Met Asp Gly Arg Ile Leu Gln Val Glu Leu Val Arg Ser Leu
   465 470 475 480
   Asn Tyr Leu Leu Tyr Val Ser Asn Phe Ser Leu Gly Asp Ser Gly Gln
   485 490 495
   Pro Val Gln Arg Asp Val Ser Arg Leu Gly Asp His Leu Leu Phe Ala
   500 505 510
   Ser Gly Asp Gln Val Phe Gln Val Pro Ile Arg Gly Pro Gly Cys Arg
   515 520 525
   His Phe Leu Thr Cys Gly Arg Cys Leu Arg Ala Trp His Phe Met Gly
   530 535 540
   Cys Gly Trp Cys Gly Asn Met Cys Gly Gln Gln Lys Glu Cys Pro Gly
   545 550 555 560
   Ser Trp Gln Gln Asp His Cys Pro Pro Lys Leu Thr Glu Phe His Pro
   565 570 575
   His Ser Gly Pro Leu Arg Gly Ser Thr Arg Leu Thr Leu Cys Gly Ser
   580 585 590
   Asn Phe Tyr Leu His Pro Ser Gly Leu Val Pro Glu Gly Thr His Gln
   595 600 605
   Val Thr Val Gly Gln Ser Pro Cys Arg Pro Leu Pro Lys Asp Ser Ser
   610 615 620
   Lys Leu Arg Pro Val Pro Arg Lys Asp Phe Val Glu Glu Phe Glu Cys
   625 630 635 640
   Glu Leu Glu Pro Leu Gly Thr Gln Ala Val Gly Pro Thr Asn Val Ser
   645 650 655
   Leu Thr Val Thr Asn Met Pro Pro Gly Lys His Phe Arg Val Asp Gly
   660 665 670
   Thr Ser Val Leu Arg Gly Phe Ser Phe Met Glu Pro Val Leu Ile Ala
   675 680 685
   Val Gln Pro Leu Phe Gly Pro Arg Ala Gly Gly Thr Cys Leu Thr Leu
   690 695 700
   Glu Gly Gln Ser Leu Ser Val Gly Thr Ser Arg Ala Val Leu Val Asn
   705 710 715 720
   Gly Thr Glu Cys Leu Leu Ala Arg Val Ser Glu Gly Gln Leu Leu Cys
   725 730 735
   Ala Thr Pro Pro Gly Ala Thr Val Ala Ser Val Pro Leu Ser Leu Gln
   740 745 750
   Val Gly Gly Ala Gln Val Pro Gly Ser Trp Thr Phe Gln Tyr Arg Glu
   755 760 765
   Asp Pro Val Val Leu Ser Ile Ser Pro Asn Cys Gly Tyr Ile Asn Ser
   770 775 780
   His Ile Thr Ile Cys Gly Gln His Leu Thr Ser Ala Trp His Leu Val
   785 790 795 800
   Leu Ser Phe His Asp Gly Leu Arg Ala Val Glu Ser Arg Cys Glu Arg
                   805                 810                 815
   Gln Leu Pro Glu Gln Gln Leu Cys Arg Leu Pro Glu Tyr Val Val Arg
               820                 825                 830
   Asp Pro Gln Gly Trp Val Ala Gly Asn Leu Ser Ala Arg Gly Asp Gly
           835                 840                 845
   Ala Ala Gly Phe Thr Leu Pro Gly Phe Arg Phe Leu Pro Pro Pro His
       850                 855                 860
   Pro Pro Ser Ala Asn Leu Val Pro Leu Lys Pro Glu Glu His Ala Ile
   865                 870                 875                 880
   Lys Phe Glu Tyr Ile Gly Leu Gly Ala Val Ala Asp Cys Val Gly Ile
                   885                 890                 895
   Asn Val Thr Val Gly Gly Glu Ser Cys Gln His Glu Phe Arg Gly Asp
               900                 905                 910
   Met Val Val Cys Pro Leu Pro Pro Ser Leu Gln Leu Gly Gln Asp Gly
           915                 920                 925
   Ala Pro Leu Gln Val Cys Val Asp Gly Glu Cys His Ile Leu Gly Arg
       930                 935                 940
   Val Val Arg Pro Gly Pro Asp Gly Val Pro Gln Ser Thr Leu Leu Gly
   945                 950                 955                 960
   Ile Leu Leu Pro Leu Leu Leu Leu Val Ala Ala Leu Ala Thr Ala Leu
                   965                 970                 975
   Val Phe Ser Tyr Trp Trp Arg Arg Lys Gln Leu Val Leu Pro Pro Asn
               980                 985                 990
   Leu Asn Asp Leu Ala Ser Leu Asp Gln Thr Ala Gly Ala Thr Pro Leu
           995                 1000                1005
   Pro Ile Leu Tyr Ser Gly Ser Asp Tyr Arg Ser Gly Leu Ala Leu
       1010                1015                1020
   Pro Ala Ile Asp Gly Leu Asp Ser Thr Thr Cys Val His Gly Ala
       1025                1030                1035
   Ser Phe Ser Asp Ser Glu Asp Glu Ser Cys Val Pro Leu Leu Arg
       1040                1045                1050
   Lys Glu Ser Ile Gln Leu Arg Asp Leu Asp Ser Ala Leu Leu Ala
   1055 1060 1065
   Glu Val Lys Asp Val Leu Ile Pro His Glu Arg Val Val Thr His
   1070 1075 1080
   Ser Asp Arg Val Ile Gly Lys Gly His Phe Gly Val Val Tyr His
   1085 1090 1095
   Gly Glu Tyr Ile Asp Gln Ala Gln Asn Arg Ile Gln Cys Ala Ile
   1100 1105 1110
   Lys Ser Leu Ser Arg Ile Thr Glu Met Gln Gln Val Glu Ala Phe
   1115 1120 1125
   Leu Arg Glu Gly Leu Leu Met Arg Gly Leu Asn His Pro Asn Val
   1130 1135 1140
   Leu Ala Leu Ile Gly Ile Met Leu Pro Pro Glu Gly Leu Pro His
   1145 1150 1155
   Val Leu Leu Pro Tyr Met Cys His Gly Asp Leu Leu Gln Phe Ile
   1160 1165 1170
   Arg Ser Pro Gln Arg Asn Pro Thr Val Lys Asp Leu Ile Ser Phe
   1175 1180 1185
   Gly Leu Gln Val Ala Arg Gly Met Glu Tyr Leu Ala Glu Gln Lys
   1190 1195 1200
   Phe Val His Arg Asp Leu Ala Ala Arg Asn Cys Met Leu Asp Glu
   1205 1210 1215
   Ser Phe Thr Val Lys Val Ala Asp Phe Gly Leu Ala Arg Asp Ile
   1220 1225 1230
   Leu Asp Arg Glu Tyr Tyr Ser Val Gln Gln His Arg His Ala Arg
   1235 1240 1245
   Leu Pro Val Lys Trp Met Ala Leu Glu Ser Leu Gln Thr Tyr Arg
   1250 1255 1260
   Phe Thr Thr Lys Ser Asp Val Trp Ser Phe Gly Val Leu Leu Trp
   1265 1270 1275
   Glu Leu Leu Thr Arg Gly Ala Pro Pro Tyr Arg His Ile Asp Pro
   1280 1285 1290
   Phe Asp Leu Thr His Phe Leu Ala Gln Gly Arg Arg Leu Pro Gln
   1295 1300 1305
   Pro Glu Tyr Cys Pro Asp Ser Leu Tyr Gln Val Met Gln Gln Cys
   1310 1315 1320
   Trp Glu Ala Asp Pro Ala Val Arg Pro Thr Phe Arg Val Leu Val
   1325 1330 1335
   Gly Glu Val Glu Gln Ile Val Ser Ala Leu Leu Gly Asp His Tyr
   1340 1345 1350
   Val Gln Leu Pro Ala Thr Tyr Met Asn Leu Gly Pro Ser Thr Ser
   1355 1360 1365
   His Glu Met Asn Val Arg Pro Glu Gln Pro Gln Phe Ser Pro Met
   1370 1375 1380
   Pro Gly Asn Val Arg Arg Pro Arg Pro Leu Ser Glu Pro Pro Arg
   1385 1390 1395
   Pro Thr
   1400
<210> 3
   <211> 4000
   <212> DNA
   <213> Homo sapiens
<400> 3
   ggatcctcta gggtcccagc tcgcctcgat ggagctcctc ccgccgctgc ctcagtcctt 60
   cctgttgctg ctgctgttgc ctgccaagcc cgcggcgggc gaggactggc agtgcccgcg 120
   caccccctac gcggcctctc gcgactttga cgtgaagtac gtggtgccca gcttctccgc 180
   cggaggcctg gtacaggcca tggtgaccta cgagggcgac agaaatgaga gtgctgtgtt 240
   tgtagccata cgcaatcgcc tgcatgtgct tgggcctgac ctgaagtctg tccagagcct 300
   ggccacgggc cctgctggag accctggctg ccagacgtgt gcagcctgtg gcccaggacc 360
   ccacggccct cccggtgaca cagacacaaa ggtgctggtg ctggatcccg cgctgcctgc 420
   gctggtcagt tgtggctcca gcctgcaggg ccgctgcttc ctgcatgacc tagagcccca 480
   agggacagcc gtgcatctgg cagcgccagc ctgcctcttc tcagcccacc ataaccggcc 540
   cgatgactgc cccgactgtg tggccagccc attgggcacc cgtgtaactg tggttgagca 600
   aggccaggcc tcctatttct acgtggcatc ctcactggac gcagccgtgg ctggcagctt 660
   cagcccacgc tcagtgtcta tcaggcgtct caaggctgac gcctcgggat tcgcaccggg 720
   ctttgtggcg ttgtcagtgc tgcccaagca tcttgtctcc tacagtattg aatacgtgca 780
   cagcttccac acgggagcct tcgtatactt cctgactgta cagccggcca gcgtgacaga 840
   tgatcctagt gccctgcaca cacgcctggc acggcttagc gccactgagc cagagttggg 900
   tgactatcgg gagctggtcc tcgactgcag atttgctcca aaacgcaggc gccggggggc 960
   cccagaaggc ggacagccct accctgtgct gcaggtggcc cactccgctc cagtgggtgc 1020
   ccaacttgcc actgagctga gcatcgccga gggccaggaa gtactatttg gggtctttgt 1080
   gactggcaag gatggtggtc ctggcgtggg ccccaactct gtcgtctgtg ccttccccat 1140
   tgacctgctg gacacactaa ttgatgaggg tgtggagcgc tgttgtgaat ccccagtcca 1200
   tccaggcctc cggcgaggcc tcgacttctt ccagtcgccc agtttttgcc ccaacccgcc 1260
   tggcctggaa gccctcagcc ccaacaccag ctgccgccac ttccctctgc tggtcagtag 1320
   cagcttctca cgtgtggacc tattcaatgg gctgttggga ccagtacagg tcactgcatt 1380
   gtatgtgaca cgccttgaca acgtcacagt ggcacacatg ggcacaatgg atgggcgtat 1440
   cctgcaggtg gagctggtca ggtcactaaa ctacttgctg tatgtgtcca acttctcact 1500
   gggtgacagt gggcagcccg tgcagcggga tgtcagtcgt cttggggacc acctactctt 1560
   tgcctctggg gaccaggttt tccaggtacc tatccgaggc cctggctgcc gccacttcct 1620
   gacctgtggg cgttgcctaa gggcatggca tttcatgggc tgtggctggt gtgggaacat 1680
   gtgcggccag cagaaggagt gtcctggctc ctggcaacag gaccactgcc cacctaagct 1740
   tactgagttc cacccccaca gtggacctct aaggggcagt acaaggctga ccctgtgtgg 1800
   ctccaacttc taccttcacc cttctggtct ggtgcctgag ggaacccatc aggtcactgt 1860
   gggccaaagt ccctgccggc cactgcccaa ggacagctca aaactcagac cagtgccccg 1920
   gaaagacttt gtagaggagt ttgagtgtga actggagccc ttgggcaccc aggcagtggg 1980
   gcctaccaac gtcagcctca ccgtgactaa catgccaccg ggcaagcact tccgggtaga 2040
   cggcacctcc gtgctgagag gcttctcttt catggagcca gtgctgatag cagtgcaacc 2100
   cctctttggc ccacgggcag gaggcacctg tctcactctt gaaggccaga gtctgtctgt 2160
   aggcaccagc cgggctgtgc tggtcaatgg gactgagtgt ctgctagcac gggtcagtga 2220
   ggggcagctt ttatgtgcca caccccctgg ggccacggtg gccagtgtcc cccttagcct 2280
   gcaggtgggg ggtgcccagg tacctggttc ctggaccttc cagtacagag aagaccctgt 2340
   cgtgctaagc atcagcccca actgtggcta catcaactcc cacatcacca tctgtggcca 2400
   gcatctaact tcagcatggc acttagtgct gtcattccat gacgggctta gggcagtgga 2460
   aagcaggtgt gagaggcagc ttccagagca gcagctgtgc cgccttcctg aatatgtggt 2520
   ccgagacccc cagggatggg tggcagggaa tctgagtgcc cgaggggatg gagctgctgg 2580
   ctttacactg cctggctttc gcttcctacc cccaccccat ccacccagtg ccaacctagt   2640
   tccactgaag cctgaggagc atgccattaa gtttgagtat attgggctgg gcgctgtggc   2700
   tgactgtgtg ggtatcaacg tgaccgtggg tggtgagagc tgccagcacg agttccgggg   2760
   ggacatggtt gtctgccccc tgcccccatc cctgcagctt ggccaggatg gtgccccatt   2820
   gcaggtctgc gtagatgcac tccctgccat tgatggtctg gattccacca cttgtgtcca   2880
   tggagcatcc ttctccgata gtgaagatga atcctgtgtg ccactgctgc ggaaagagtc   2940
   catccagcta agggacctgg actctgcgct cttggctgag gtcaaggatg tgctgattcc   3000
   ccatgagcgg gtggtcaccc acagtgaccg agtcattggc aaaggccact ttggagttgt   3060
   ctaccacgga gaatacatag accaggccca gaatcgaatc caatgtgcca tcaagtcact   3120
   aagtcgcatc acagagatgc agcaggtgga ggccttcctg cgagaggggc tgctcatgcg   3180
   tggcctgaac cacccgaatg tgctggctct cattggtatc atgttgccac ctgagggcct   3240
   gccccatgtg ctgctgccct atatgtgcca cggtgacctg ctccagttca tccgctcacc   3300
   tcagcggaac cccaccgtga aggacctcat cagctttggc ctgcaggtag cccgcggcat   3360
   ggagtacctg gcagagcaga agtttgtgca cagggacctg gctgcgcgga actgcatgct   3420
   ggacgagtca ttcacagtca aggtggctga ctttggtttg gcccgcgaca tcctggacag   3480
   ggagtactat agtgttcaac agcatcgcca cgctcgccta cctgtgaagt ggatggcgct   3540
   ggagagcctg cagacctata gatttaccac caagtctgat gtggtaccaa gtgatgcagc   3600
   aatgctggga ggcagaccca gcagtgcgac ccaccttcag agtactagtg ggggaggtgg   3660
   agcagatagt gtctgcactg cttggggacc attatgtgca gctgccagca acctacatga   3720
   acttgagcta accccaaggc tgcctctggg ccatgccagg ccagagcagt ggccctccac   3780
   cttgttcctg ccctttaact ttcagaggca ataggtaaat gggcccatta ggtccctcac   3840
   tccacagagt gagccagtga gggcagtcct gcaacatgta tttatggagt gcctgctgtg   3900
   gaccctgtct tctgggcaca gtggactcag cagtgaccac accaacactg acccttgaac   3960
   caataaagga acaaatgact attaaagcac aaaaaaaaaa                         4000
<210> 4
   <211> 1261
   <212> PRT
   <213> Homo sapiens
<400> 4
   Met Glu Leu Leu Pro Pro Leu Pro Gln Ser Phe Leu Leu Leu Leu Leu
   1               5                   10                  15
   Leu Pro Ala Lys Pro Ala Ala Gly Glu Asp Trp Gln Cys Pro Arg Thr
               20                  25                  30
   Pro Tyr Ala Ala Ser Arg Asp Phe Asp Val Lys Tyr Val Val Pro Ser
   35 40 45
   Phe Ser Ala Gly Gly Leu Val Gln Ala Met Val Thr Tyr Glu Gly Asp
   50 55 60
   Arg Asn Glu Ser Ala Val Phe Val Ala Ile Arg Asn Arg Leu His Val
   65 70 75 80
   Leu Gly Pro Asp Leu Lys Ser Val Gln Ser Leu Ala Thr Gly Pro Ala
   85 90 95
   Gly Asp Pro Gly Cys Gln Thr Cys Ala Ala Cys Gly Pro Gly Pro His
   100 105 110
   Gly Pro Pro Gly Asp Thr Asp Thr Lys Val Leu Val Leu Asp Pro Ala
   115 120 125
   Leu Pro Ala Leu Val Ser Cys Gly Ser Ser Leu Gln Gly Arg Cys Phe
   130 135 140
   Leu His Asp Leu Glu Pro Gln Gly Thr Ala Val His Leu Ala Ala Pro
   145 150 155 160
   Ala Cys Leu Phe Ser Ala His His Asn Arg Pro Asp Asp Cys Pro Asp
   165 170 175
   Cys Val Ala Ser Pro Leu Gly Thr Arg Val Thr Val Val Glu Gln Gly
   180 185 190
   Gln Ala Ser Tyr Phe Tyr Val Ala Ser Ser Leu Asp Ala Ala Val Ala
   195 200 205
   Gly Ser Phe Ser Pro Arg Ser Val Ser Ile Arg Arg Leu Lys Ala Asp
   210 215 220
   Ala Ser Gly Phe Ala Pro Gly Phe Val Ala Leu Ser Val Leu Pro Lys
   225 230 235 240
   His Leu Val Ser Tyr Ser Ile Glu Tyr Val His Ser Phe His Thr Gly
   245 250 255
   Ala Phe Val Tyr Phe Leu Thr Val Gln Pro Ala Ser Val Thr Asp Asp
   260 265 270
   Pro Ser Ala Leu His Thr Arg Leu Ala Arg Leu Ser Ala Thr Glu Pro
   275 280 285
   Glu Leu Gly Asp Tyr Arg Glu Leu Val Leu Asp Cys Arg Phe Ala Pro
   290 295 300
   Lys Arg Arg Arg Arg Gly Ala Pro Glu Gly Gly Gln Pro Tyr Pro Val
   305 310 315 320
   Leu Gln Val Ala His Ser Ala Pro Val Gly Ala Gln Leu Ala Thr Glu
   325 330 335
   Leu Ser Ile Ala Glu Gly Gln Glu Val Leu Phe Gly Val Phe Val Thr
   340 345 350
   Gly Lys Asp Gly Gly Pro Gly Val Gly Pro Asn Ser Val Val Cys Ala
   355 360 365
   Phe Pro Ile Asp Leu Leu Asp Thr Leu Ile Asp Glu Gly Val Glu Arg
   370 375 380
   Cys Cys Glu Ser Pro Val His Pro Gly Leu Arg Arg Gly Leu Asp Phe
   385 390 395 400
   Phe Gln Ser Pro Ser Phe Cys Pro Asn Pro Pro Gly Leu Glu Ala Leu
   405 410 415
   Ser Pro Asn Thr Ser Cys Arg His Phe Pro Leu Leu Val Ser Ser Ser
   420 425 430
   Phe Ser Arg Val Asp Leu Phe Asn Gly Leu Leu Gly Pro Val Gln Val
   435 440 445
   Thr Ala Leu Tyr Val Thr Arg Leu Asp Asn Val Thr Val Ala His Met
   450 455 460
   Gly Thr Met Asp Gly Arg Ile Leu Gln Val Glu Leu Val Arg Ser Leu
   465 470 475 480
   Asn Tyr Leu Leu Tyr Val Ser Asn Phe Ser Leu Gly Asp Ser Gly Gln
   485 490 495
   Pro Val Gln Arg Asp Val Ser Arg Leu Gly Asp His Leu Leu Phe Ala
   500 505 510
   Ser Gly Asp Gln Val Phe Gln Val Pro Ile Arg Gly Pro Gly Cys Arg
   515 520 525
   His Phe Leu Thr Cys Gly Arg Cys Leu Arg Ala Trp His Phe Met Gly
   530 535 540
   Cys Gly Trp Cys Gly Asn Met Cys Gly Gln Gln Lys Glu Cys Pro Gly
   545 550 555 560
   Ser Trp Gln Gln Asp His Cys Pro Pro Lys Leu Thr Glu Phe His Pro
   565 570 575
   His Ser Gly Pro Leu Arg Gly Ser Thr Arg Leu Thr Leu Cys Gly Ser
   580 585 590
   Asn Phe Tyr Leu His Pro Ser Gly Leu Val Pro Glu Gly Thr His Gln
   595 600 605
   Val Thr Val Gly Gln Ser Pro Cys Arg Pro Leu Pro Lys Asp Ser Ser
   610 615 620
   Lys Leu Arg Pro Val Pro Arg Lys Asp Phe Val Glu Glu Phe Glu Cys
   625 630 635 640
   Glu Leu Glu Pro Leu Gly Thr Gln Ala Val Gly Pro Thr Asn Val Ser
   645 650 655
   Leu Thr Val Thr Asn Met Pro Pro Gly Lys His Phe Arg Val Asp Gly
   660 665 670
   Thr Ser Val Leu Arg Gly Phe Ser Phe Met Glu Pro Val Leu Ile Ala
   675 680 685
   Val Gln Pro Leu Phe Gly Pro Arg Ala Gly Gly Thr Cys Leu Thr Leu
   690 695 700
   Glu Gly Gln Ser Leu Ser Val Gly Thr Ser Arg Ala Val Leu Val Asn
   705 710 715 720
   Gly Thr Glu Cys Leu Leu Ala Arg Val Ser Glu Gly Gln Leu Leu Cys
   725 730 735
   Ala Thr Pro Pro Gly Ala Thr Val Ala Ser Val Pro Leu Ser Leu Gln
   740 745 750
   Val Gly Gly Ala Gln Val Pro Gly Ser Trp Thr Phe Gln Tyr Arg Glu
   755 760 765
   Asp Pro Val Val Leu Ser Ile Ser Pro Asn Cys Gly Tyr Ile Asn Ser
   770 775 780
   His Ile Thr Ile Cys Gly Gln His Leu Thr Ser Ala Trp His Leu Val
   785 790 795 800
   Leu Ser Phe His Asp Gly Leu Arg Ala Val Glu Ser Arg Cys Glu Arg
   805 810 815
   Gln Leu Pro Glu Gln Gln Leu Cys Arg Leu Pro Glu Tyr Val Val Arg
   820 825 830
   Asp Pro Gln Gly Trp Val Ala Gly Asn Leu Ser Ala Arg Gly Asp Gly
   835 840 845
   Ala Ala Gly Phe Thr Leu Pro Gly Phe Arg Phe Leu Pro Pro Pro His
   850 855 860
   Pro Pro Ser Ala Asn Leu Val Pro Leu Lys Pro Glu Glu His Ala Ile
   865 870 875 880
   Lys Phe Glu Tyr Ile Gly Leu Gly Ala Val Ala Asp Cys Val Gly Ile
   885 890 895
   Asn Val Thr Val Gly Gly Glu Ser Cys Gln His Glu Phe Arg Gly Asp
   900 905 910
   Met Val Val Cys Pro Leu Pro Pro Ser Leu Gln Leu Gly Gln Asp Gly
   915 920 925
   Ala Pro Leu Gln Val Cys Val Asp Ala Leu Pro Ala Ile Asp Gly Leu
   930 935 940
   Asp Ser Thr Thr Cys Val His Gly Ala Ser Phe Ser Asp Ser Glu Asp
   945 950 955 960
   Glu Ser Cys Val Pro Leu Leu Arg Lys Glu Ser Ile Gln Leu Arg Asp
   965 970 975
   Leu Asp Ser Ala Leu Leu Ala Glu Val Lys Asp Val Leu Ile Pro His
   980 985 990
   Glu Arg Val Val Thr His Ser Asp Arg Val Ile Gly Lys Gly His Phe
   995 1000 1005
   Gly Val Val Tyr His Gly Glu Tyr Ile Asp Gln Ala Gln Asn Arg
   1010 1015 1020
   Ile Gln Cys Ala Ile Lys Ser Leu Ser Arg Ile Thr Glu Met Gln
   1025 1030 1035
   Gln Val Glu Ala Phe Leu Arg Glu Gly Leu Leu Met Arg Gly Leu
   1040 1045 1050
   Asn His Pro Asn Val Leu Ala Leu Ile Gly Ile Met Leu Pro Pro
   1055 1060 1065
   Glu Gly Leu Pro His Val Leu Leu Pro Tyr Met Cys His Gly Asp
   1070 1075 1080
   Leu Leu Gln Phe Ile Arg Ser Pro Gln Arg Asn Pro Thr Val Lys
   1085 1090 1095
   Asp Leu Ile Ser Phe Gly Leu Gln Val Ala Arg Gly Met Glu Tyr
   1100 1105 1110
   Leu Ala Glu Gln Lys Phe Val His Arg Asp Leu Ala Ala Arg Asn
   1115 1120 1125
   Cys Met Leu Asp Glu Ser Phe Thr Val Lys Val Ala Asp Phe Gly
   1130 1135 1140
   Leu Ala Arg Asp Ile Leu Asp Arg Glu Tyr Tyr Ser Val Gln Gln
   1145 1150 1155
   His Arg His Ala Arg Leu Pro Val Lys Trp Met Ala Leu Glu Ser
   1160 1165 1170
   Leu Gln Thr Tyr Arg Phe Thr Thr Lys Ser Asp Val Val Pro Ser
   1175 1180 1185
   Asp Ala Ala Met Leu Gly Gly Arg Pro Ser Ser Ala Thr His Leu
   1190 1195 1200
   Gln Ser Thr Ser Gly Gly Gly Gly Ala Asp Ser Val Cys Thr Ala
   1205 1210 1215
   Trp Gly Pro Leu Cys Ala Ala Ala Ser Asn Leu His Glu Leu Glu
   1220 1225 1230
   Leu Thr Pro Arg Leu Pro Leu Gly His Ala Arg Pro Glu Gln Trp
   1235 1240 1245
   Pro Ser Thr Leu Phe Leu Pro Phe Asn Phe Gln Arg Gln
   1250 1255 1260
<210> 5
   <211> 3880
   <212> DNA
   <213> Homo sapiens
<400> 5
   ggatcctcta gggtcccagc tcgcctcgat ggagctcctc ccgccgctgc ctcagtcctt     60
   cctgttgctg ctgctgttgc ctgccaagcc cgcggcgggc gaggactggc agtgcccgcg    120
   caccccctac gcggcctctc gcgactttga cgtgaagtac gtggtgccca gcttctccgc    180
   cggaggcctg gtacaggcca tggtgaccta cgagggcgac agaaatgaga gtgctgtgtt    240
   tgtagccata cgcaatcgcc tgcatgtgct tgggcctgac ctgaagtctg tccagagcct    300
   ggccacgggc cctgctggag accctggctg ccagacgtgt gcagcctgtg gcccaggacc    360
   ccacggccct cccggtgaca cagacacaaa ggtgctggtg ctggatcccg cgctgcctgc    420
   gctggtcagt tgtggctcca gcctgcaggg ccgctgcttc ctgcatgacc tagagcccca    480
   agggacagcc gtgcatctgg cagcgccagc ctgcctcttc tcagcccacc ataaccggcc    540
   cgatgactgc cccgactgtg tggccagccc attgggcacc cgtgtaactg tggttgagca    600
   aggccaggcc tcctatttct acgtggcatc ctcactggac gcagccgtgg ctggcagctt    660
   cagcccacgc tcagtgtcta tcaggcgtct caaggctgac gcctcgggat tcgcaccggg    720
   ctttgtggcg ttgtcagtgc tgcccaagca tcttgtctcc tacagtattg aatacgtgca    780
   cagcttccac acgggagcct tcgtatactt cctgactgta cagccggcca gcgtgacaga    840
   tgatcctagt gccctgcaca cacgcctggc acggcttagc gccactgagc cagagttggg    900
   tgactatcgg gagctggtcc tcgactgcag atttgctcca aaacgcaggc gccggggggc     960
   cccagaaggc ggacagccct accctgtgct gcaggtggcc cactccgctc cagtgggtgc   1020
   ccaacttgcc actgagctga gcatcgccga gggccaggaa gtactatttg gggtctttgt    1080
   gactggcaag gatggtggtc ctggcgtggg ccccaactct gtcgtctgtg ccttccccat   1140
   tgacctgctg gacacactaa ttgatgaggg tgtggagcgc tgttgtgaat ccccagtcca   1200
   tccaggcctc cggcgaggcc tcgacttctt ccagtcgccc agtttttgcc ccaacccgcc   1260
   tggcctggaa gccctcagcc ccaacaccag ctgccgccac ttccctctgc tggtcagtag   1320
   cagcttctca cgtgtggacc tattcaatgg gctgttggga ccagtacagg tcactgcatt   1380
   gtatgtgaca cgccttgaca acgtcacagt ggcacacatg ggcacaatgg atgggcgtat   1440
   cctgcaggtg gagctggtca ggtcactaaa ctacttgctg tatgtgtcca acttctcact   1500
   gggtgacagt gggcagcccg tgcagcggga tgtcagtcgt cttggggacc acctactctt   1560
   tgcctctggg gaccaggttt tccaggtacc tatccgaggc cctggctgcc gccacttcct   1620
   gacctgtggg cgttgcctaa gggcatggca tttcatgggc tgtggctggt gtgggaacat   1680
   gtgcggccag cagaaggagt gtcctggctc ctggcaacag gaccactgcc cacctaagct   1740
   tactgagttc cacccccaca gtggacctct aaggggcagt acaaggctga ccctgtgtgg   1800
   ctccaacttc taccttcacc cttctggtct ggtgcctgag ggaacccatc aggtcactgt   1860
   gggccaaagt ccctgccggc cactgcccaa ggacagctca aaactcagac cagtgccccg   1920
   gaaagacttt gtagaggagt ttgagtgtga actggagccc ttgggcaccc aggcagtggg   1980
   gcctaccaac gtcagcctca ccgtgactaa catgccaccg ggcaagcact tccgggtaga   2040
   cggcacctcc gtgctgagag gcttctcttt catggagcca gtgctgatag cagtgcaacc   2100
   cctctttggc ccacgggcag gaggcacctg tctcactctt gaaggccaga gtctgtctgt   2160
   aggcaccagc cgggctgtgc tggtcaatgg gactgagtgt ctgctagcac gggtcagtga   2220
   ggggcagctt ttatgtgcca caccccctgg ggccacggtg gccagtgtcc cccttagcct   2280
   gcaggtgggg ggtgcccagg tacctggttc ctggaccttc cagtacagag aagaccctgt   2340
   cgtgctaagc atcagcccca actgtggcta catcaactcc cacatcacca tctgtggcca   2400
   gcatctaact tcagcatggc acttagtgct gtcattccat gacgggctta gggcagtgga   2460
   aagcaggtgt gagaggcagc ttccagagca gcagctgtgc cgccttcctg aatatgtggt   2520
   ccgagacccc cagggatggg tggcagggaa tctgagtgcc cgaggggatg gagctgctgg   2580
   ctttacactg cctggctttc gcttcctacc cccaccccat ccacccagtg ccaacctagt   2640
   tccactgaag cctgaggagc atgccattaa gtttgagctt ggccaggatg gtgccccatt   2700
   gcaggtctgc gtagatgcac tccctgccat tgatggtctg gattccacca cttgtgtcca   2760
   tggagcatcc ttctccgata gtgaagatga atcctgtgtg ccactgctgc ggaaagagtc   2820
   catccagcta agggacctgg actctgcgct cttggctgag gtcaaggatg tgctgattcc   2880
   ccatgagcgg gtggtcaccc acagtgaccg agtcattggc aaaggccact ttggagttgt   2940
   ctaccacgga gaatacatag accaggccca gaatcgaatc caatgtgcca tcaagtcact   3000
   aagtcgcatc acagagatgc agcaggtgga ggccttcctg cgagaggggc tgctcatgcg   3060
   tggcctgaac cacccgaatg tgctggctct cattggtatc atgttgccac ctgagggcct   3120
   gccccatgtg ctgctgccct atatgtgcca cggtgacctg ctccagttca tccgctcacc   3180
   tcagcggaac cccaccgtga aggacctcat cagctttggc ctgcaggtag cccgcggcat   3240
   ggagtacctg gcagagcaga agtttgtgca cagggacctg gctgcgcgga actgcatgct   3300
   ggacgagtca ttcacagtca aggtggctga ctttggtttg gcccgcgaca tcctggacag   3360
   ggagtactat agtgttcaac agcatcgcca cgctcgccta cctgtgaagt ggatggcgct   3420
   ggagagcctg cagacctata gatttaccac caagtctgat gtggtaccaa gtgatgcagc   3480
   aatgctggga ggcagaccca gcagtgcgac ccaccttcag agtactagtg ggggaggtgg   3540
   agcagatagt gtctgcactg cttggggacc attatgtgca gctgccagca acctacatga   3600
   acttgagcta accccaaggc tgcctctggg ccatgccagg ccagagcagt ggccctccac   3660
   cttgttcctg ccctttaact ttcagaggca ataggtaaat gggcccatta ggtccctcac 3720
   tccacagagt gagccagtga gggcagtcct gcaacatgta tttatggagt gcctgctgtg 3780
   gaccctgtct tctgggcaca gtggactcag cagtgaccac accaacactg acccttgaac 3840
   caataaagga acaaatgact attaaagcac aaaaaaaaaa 3880
<210> 6
   <211> 1221
   <212> PRT
   <213> Homo sapiens
<400> 6
   Met Glu Leu Leu Pro Pro Leu Pro Gln Ser Phe Leu Leu Leu Leu Leu
   1               5                   10                  15
   Leu Pro Ala Lys Pro Ala Ala Gly Glu Asp Trp Gln Cys Pro Arg Thr
               20                  25                  30
   Pro Tyr Ala Ala Ser Arg Asp Phe Asp Val Lys Tyr Val Val Pro Ser
           35                  40                  45
   Phe Ser Ala Gly Gly Leu Val Gln Ala Met Val Thr Tyr Glu Gly Asp
       50                  55                  60
   Arg Asn Glu Ser Ala Val Phe Val Ala Ile Arg Asn Arg Leu His Val
   65                  70                  75                  80
   Leu Gly Pro Asp Leu Lys Ser Val Gln Ser Leu Ala Thr Gly Pro Ala
                   85                  90                  95
   Gly Asp Pro Gly Cys Gln Thr Cys Ala Ala Cys Gly Pro Gly Pro His
               100                 105                 110
   Gly Pro Pro Gly Asp Thr Asp Thr Lys Val Leu Val Leu Asp Pro Ala
           115                 120                 125
   Leu Pro Ala Leu Val Ser Cys Gly Ser Ser Leu Gln Gly Arg Cys Phe
       130                 135                 140
   Leu His Asp Leu Glu Pro Gln Gly Thr Ala Val His Leu Ala Ala Pro
   145                 150                 155                 160
   Ala Cys Leu Phe Ser Ala His His Asn Arg Pro Asp Asp Cys Pro Asp
                   165                 170                 175
   Cys Val Ala Ser Pro Leu Gly Thr Arg Val Thr Val Val Glu Gln Gly
               180                 185                 190
   Gln Ala Ser Tyr Phe Tyr Val Ala Ser Ser Leu Asp Ala Ala Val Ala
   195 200 205
   Gly Ser Phe Ser Pro Arg Ser Val Ser Ile Arg Arg Leu Lys Ala Asp
   210 215 220
   Ala Ser Gly Phe Ala Pro Gly Phe Val Ala Leu Ser Val Leu Pro Lys
   225 230 235 240
   His Leu Val Ser Tyr Ser Ile Glu Tyr Val His Ser Phe His Thr Gly
   245 250 255
   Ala Phe Val Tyr Phe Leu Thr Val Gln Pro Ala Ser Val Thr Asp Asp
   260 265 270
   Pro Ser Ala Leu His Thr Arg Leu Ala Arg Leu Ser Ala Thr Glu Pro
   275 280 285
   Glu Leu Gly Asp Tyr Arg Glu Leu Val Leu Asp Cys Arg Phe Ala Pro
   290 295 300
   Lys Arg Arg Arg Arg Gly Ala Pro Glu Gly Gly Gln Pro Tyr Pro Val
   305 310 315 320
   Leu Gln Val Ala His Ser Ala Pro Val Gly Ala Gln Leu Ala Thr Glu
   325 330 335
   Leu Ser Ile Ala Glu Gly Gln Glu Val Leu Phe Gly Val Phe Val Thr
   340 345 350
   Gly Lys Asp Gly Gly Pro Gly Val Gly Pro Asn Ser Val Val Cys Ala
   355 360 365
   Phe Pro Ile Asp Leu Leu Asp Thr Leu Ile Asp Glu Gly Val Glu Arg
   370 375 380
   Cys Cys Glu Ser Pro Val His Pro Gly Leu Arg Arg Gly Leu Asp Phe
   385 390 395 400
   Phe Gln Ser Pro Ser Phe Cys Pro Asn Pro Pro Gly Leu Glu Ala Leu
   405 410 415
   Ser Pro Asn Thr Ser Cys Arg His Phe Pro Leu Leu Val Ser Ser Ser
   420 425 430
   Phe Ser Arg Val Asp Leu Phe Asn Gly Leu Leu Gly Pro Val Gln Val
               435                 440                 445
   Thr Ala Leu Tyr Val Thr Arg Leu Asp Asn Val Thr Val Ala His Met
       450                 455                 460
   Gly Thr Met Asp Gly Arg Ile Leu Gln Val Glu Leu Val Arg Ser Leu
   465                 470                 475                 480
   Asn Tyr Leu Leu Tyr Val Ser Asn Phe Ser Leu Gly Asp Ser Gly Gln
                   485                 490                 495
   Pro Val Gln Arg Asp Val Ser Arg Leu Gly Asp His Leu Leu Phe Ala
               500                 505                 510
   Ser Gly Asp Gln Val Phe Gln Val Pro Ile Arg Gly Pro Gly Cys Arg
           515                 520                 525
   His Phe Leu Thr Cys Gly Arg Cys Leu Arg Ala Trp His Phe Met Gly
       530                 535                 540
   Cys Gly Trp Cys Gly Asn Met Cys Gly Gln Gln Lys Glu Cys Pro Gly
   545                 550                 555                 560
   Ser Trp Gln Gln Asp His Cys Pro Pro Lys Leu Thr Glu Phe His Pro
                   565                 570                 575
   His Ser Gly Pro Leu Arg Gly Ser Thr Arg Leu Thr Leu Cys Gly Ser
               580                 585                 590
   Asn Phe Tyr Leu His Pro Ser Gly Leu Val Pro Glu Gly Thr His Gln
           595                 600                 605
   Val Thr Val Gly Gln Ser Pro Cys Arg Pro Leu Pro Lys Asp Ser Ser
       610                 615                 620
   Lys Leu Arg Pro Val Pro Arg Lys Asp Phe Val Glu Glu Phe Glu Cys
   625                 630                 635                 640
   Glu Leu Glu Pro Leu Gly Thr Gln Ala Val Gly Pro Thr Asn Val Ser
                   645                 650                 655
   Leu Thr Val Thr Asn Met Pro Pro Gly Lys His Phe Arg Val Asp Gly
               660                 665                 670
   Thr Ser Val Leu Arg Gly Phe Ser Phe Met Glu Pro Val Leu Ile Ala
           675                 680                 685
   Val Gln Pro Leu Phe Gly Pro Arg Ala Gly Gly Thr Cys Leu Thr Leu
   690 695 700
   Glu Gly Gln Ser Leu Ser Val Gly Thr Ser Arg Ala Val Leu Val Asn
   705 710 715 720
   Gly Thr Glu Cys Leu Leu Ala Arg Val Ser Glu Gly Gln Leu Leu Cys
   725 730 735
   Ala Thr Pro Pro Gly Ala Thr Val Ala Ser Val Pro Leu Ser Leu Gln
   740 745 750
   Val Gly Gly Ala Gln Val Pro Gly Ser Trp Thr Phe Gln Tyr Arg Glu
   755 760 765
   Asp Pro Val Val Leu Ser Ile Ser Pro Asn Cys Gly Tyr Ile Asn Ser
   770 775 780
   His Ile Thr Ile Cys Gly Gln His Leu Thr Ser Ala Trp His Leu Val
   785 790 795 800
   Leu Ser Phe His Asp Gly Leu Arg Ala Val Glu Ser Arg Cys Glu Arg
   805 810 815
   Gln Leu Pro Glu Gln Gln Leu Cys Arg Leu Pro Glu Tyr Val Val Arg
   820 825 830
   Asp Pro Gln Gly Trp Val Ala Gly Asn Leu Ser Ala Arg Gly Asp Gly
   835 840 845
   Ala Ala Gly Phe Thr Leu Pro Gly Phe Arg Phe Leu Pro Pro Pro His
   850 855 860
   Pro Pro Ser Ala Asn Leu Val Pro Leu Lys Pro Glu Glu His Ala Ile
   865 870 875 880
   Lys Phe Glu Leu Gly Gln Asp Gly Ala Pro Leu Gln Val Cys Val Asp
   885 890 895
   Ala Leu Pro Ala Ile Asp Gly Leu Asp Ser Thr Thr Cys Val His Gly
   900 905 910
   Ala Ser Phe Ser Asp Ser Glu Asp Glu Ser Cys Val Pro Leu Leu Arg
   915 920 925
   Lys Glu Ser Ile Gln Leu Arg Asp Leu Asp Ser Ala Leu Leu Ala Glu
   930 935 940
   Val Lys Asp Val Leu Ile Pro His Glu Arg Val Val Thr His Ser Asp
   945                 950                 955                 960
   Arg Val Ile Gly Lys Gly His Phe Gly Val Val Tyr His Gly Glu Tyr
                   965                 970                 975
   Ile Asp Gln Ala Gln Asn Arg Ile Gln Cys Ala Ile Lys Ser Leu Ser
               980                 985                 990
   Arg Ile Thr Glu Met Gln Gln Val Glu Ala Phe Leu Arg Glu Gly Leu
           995                 1000                1005
   Leu Met Arg Gly Leu Asn His Pro Asn Val Leu Ala Leu Ile Gly
       1010                1015                1020
   Ile Met Leu Pro Pro Glu Gly Leu Pro His Val Leu Leu Pro Tyr
       1025                1030                1035
   Met Cys His Gly Asp Leu Leu Gln Phe Ile Arg Ser Pro Gln Arg
       1040                1045                1050
   Asn Pro Thr Val Lys Asp Leu Ile Ser Phe Gly Leu Gln Val Ala
       1055                1060                1065
   Arg Gly Met Glu Tyr Leu Ala Glu Gln Lys Phe Val His Arg Asp
       1070                1075                1080
   Leu Ala Ala Arg Asn Cys Met Leu Asp Glu Ser Phe Thr Val Lys
       1085                1090                1095
   Val Ala Asp Phe Gly Leu Ala Arg Asp Ile Leu Asp Arg Glu Tyr
       1100                1105                1110
   Tyr Ser Val Gln Gln His Arg His Ala Arg Leu Pro Val Lys Trp
       1115                1120                1125
   Met Ala Leu Glu Ser Leu Gln Thr Tyr Arg Phe Thr Thr Lys Ser
       1130                1135                1140
   Asp Val Val Pro Ser Asp Ala Ala Met Leu Gly Gly Arg Pro Ser
       1145                1150                1155
   Ser Ala Thr His Leu Gln Ser Thr Ser Gly Gly Gly Gly Ala Asp
       1160                1165                1170
   Ser Val Cys Thr Ala Trp Gly Pro Leu Cys Ala Ala Ala Ser Asn
       1175                1180                1185
   Leu His Glu Leu Glu Leu Thr Pro Arg Leu Pro Leu Gly His Ala
   1190 1195 1200
   Arg Pro Glu Gln Trp Pro Ser Thr Leu Phe Leu Pro Phe Asn Phe
   1205 1210 1215
   Gln Arg Gln
   1220
<210> 7
   <211> 3853
   <212> DNA
   <213> Homo sapiens
<400> 7
<210> 8
   <211> 1212
   <212> PRT
   <213> Homo sapiens
<400> 8
   Met Glu Leu Leu Pro Pro Leu Pro Gln Ser Phe Leu Leu Leu Leu Leu
   1               5                   10                  15
   Leu Pro Ala Lys Pro Ala Ala Gly Glu Asp Trp Gln Cys Pro Arg Thr
               20                  25                  30
   Pro Tyr Ala Ala Ser Arg Asp Phe Asp Val Lys Tyr Val Val Pro Ser
           35                  40                  45
   Phe Ser Ala Gly Gly Leu Val Gln Ala Met Val Thr Tyr Glu Gly Asp
       50                  55                  60
   Arg Asn Glu Ser Ala Val Phe Val Ala Ile Arg Asn Arg Leu His Val
   65                  70                  75                  80
   Leu Gly Pro Asp Leu Lys Ser Val Gln Ser Leu Ala Thr Gly Pro Ala
                   85                  90                  95
   Gly Asp Pro Gly Cys Gln Thr Cys Ala Ala Cys Gly Pro Gly Pro His
               100                 105                 110
   Gly Pro Pro Gly Asp Thr Asp Thr Lys Val Leu Val Leu Asp Pro Ala
           115                 120                 125
   Leu Pro Ala Leu Val Ser Cys Gly Ser Ser Leu Gln Gly Arg Cys Phe
       130                     135                     140
   Leu His Asp Leu Glu Pro Gln Gly Thr Ala Val His Leu Ala Ala Pro
   145                     150                     155                     160
   Ala Cys Leu Phe Ser Ala His His Asn Arg Pro Asp Asp Cys Pro Asp
                       165                     170                     175
   Cys Val Ala Ser Pro Leu Gly Thr Arg Val Thr Val Val Glu Gln Gly
                   180                     185                     190
   Gln Ala Ser Tyr Phe Tyr Val Ala Ser Ser Leu Asp Ala Ala Val Ala
               195                     200                     205
   Gly Ser Phe Ser Pro Arg Ser Val Ser Ile Arg Arg Leu Lys Ala Asp
       210                     215                     220
   Ala Ser Gly Phe Ala Pro Gly Phe Val Ala Leu Ser Val Leu Pro Lys
   225                     230                     235                     240
   His Leu Val Ser Tyr Ser Ile Glu Tyr Val His Ser Phe His Thr Gly
                       245                     250                     255
   Ala Phe Val Tyr Phe Leu Thr Val Gln Pro Ala Ser Val Thr Asp Asp
                   260                     265                     270
   Pro Ser Ala Leu His Thr Arg Leu Ala Arg Leu Ser Ala Thr Glu Pro
               275                     280                     285
   Glu Leu Gly Asp Tyr Arg Glu Leu Val Leu Asp Cys Arg Phe Ala Pro
       290                     295                     300
   Lys Arg Arg Arg Arg Gly Ala Pro Glu Gly Gly Gln Pro Tyr Pro Val
   305                     310                     315                     320
   Leu Gln Val Ala His Ser Ala Pro Val Gly Ala Gln Leu Ala Thr Glu
                       325                     330                     335
   Leu Ser Ile Ala Glu Gly Gln Glu Val Leu Phe Gly Val Phe Val Thr
                   340                     345                     350
   Gly Lys Asp Gly Gly Pro Gly Val Gly Pro Asn Ser Val Val Cys Ala
               355                     360                     365
   Phe Pro Ile Asp Leu Leu Asp Thr Leu Ile Asp Glu Gly Val Glu Arg
       370                     375                     380
   Cys Cys Glu Ser Pro Val His Pro Gly Leu Arg Arg Gly Leu Asp Phe
   385 390 395 400
   Phe Gln Ser Pro Ser Phe Cys Pro Asn Pro Pro Gly Leu Glu Ala Leu
   405 410 415
   Ser Pro Asn Thr Ser Cys Arg His Phe Pro Leu Leu Val Ser Ser Ser
   420 425 430
   Phe Ser Arg Val Asp Leu Phe Asn Gly Leu Leu Gly Pro Val Gln Val
   435 440 445
   Thr Ala Leu Tyr Val Thr Arg Leu Asp Asn Val Thr Val Ala His Met
   450 455 460
   Gly Thr Met Asp Gly Arg Ile Leu Gln Val Glu Leu Val Arg Ser Leu
   465 470 475 480
   Asn Tyr Leu Leu Tyr Val Ser Asn Phe Ser Leu Gly Asp Ser Gly Gln
   485 490 495
   Pro Val Gln Arg Asp Val Ser Arg Leu Gly Asp His Leu Leu Phe Ala
   500 505 510
   Ser Gly Asp Gln Val Phe Gln Val Pro Ile Arg Gly Pro Gly Cys Arg
   515 520 525
   His Phe Leu Thr Cys Gly Arg Cys Leu Arg Ala Trp His Phe Met Gly
   530 535 540
   Cys Gly Trp Cys Gly Asn Met Cys Gly Gln Gln Lys Glu Cys Pro Gly
   545 550 555 560
   Ser Trp Gln Gln Asp His Cys Pro Pro Lys Leu Thr Glu Phe His Pro
   565 570 575
   His Ser Gly Pro Leu Arg Gly Ser Thr Arg Leu Thr Leu Cys Gly Ser
   580 585 590
   Asn Phe Tyr Leu His Pro Ser Gly Leu Val Pro Glu Gly Thr His Gln
   595 600 605
   Val Thr Val Gly Gln Ser Pro Cys Arg Pro Leu Pro Lys Asp Ser Ser
   610 615 620
   Lys Leu Arg Pro Val Pro Arg Lys Asp Phe Val Glu Glu Phe Glu Cys
   625                 630                 635                 640
   Glu Leu Glu Pro Leu Gly Thr Gln Ala Val Gly Pro Thr Asn Val Ser
                   645                 650                 655
   Leu Thr Val Thr Asn Met Pro Pro Gly Lys His Phe Arg Val Asp Gly
               660                 665                 670
   Thr Ser Val Leu Arg Gly Phe Ser Phe Met Glu Pro Val Leu Ile Ala
           675                 680                 685
   Val Gln Pro Leu Phe Gly Pro Arg Ala Gly Gly Thr Cys Leu Thr Leu
       690                 695                 700
   Glu Gly Gln Ser Leu Ser Val Gly Thr Ser Arg Ala Val Leu Val Asn
   705                 710                 715                 720
   Gly Thr Glu Cys Leu Leu Ala Arg Val Ser Glu Gly Gln Leu Leu Cys
                   725                 730                 735
   Ala Thr Pro Pro Gly Ala Thr Val Ala Ser Val Pro Leu Ser Leu Gln
               740                 745                 750
   Val Gly Gly Ala Gln Val Pro Gly Ser Trp Thr Phe Gln Tyr Arg Glu
           755                 760                 765
   Asp Pro Val Val Leu Ser Ile Ser Pro Asn Cys Gly Tyr Ile Asn Ser
       770                 775                 780
   His Ile Thr Ile Cys Gly Gln His Leu Thr Ser Ala Trp His Leu Val
   785                 790                 795                 800
   Leu Ser Phe His Asp Gly Leu Arg Ala Val Glu Ser Arg Cys Glu Arg
                   805                 810                 815
   Gln Leu Pro Glu Gln Gln Leu Cys Arg Leu Pro Glu Tyr Val Val Arg
               820                 825                 830
   Asp Pro Gln Gly Trp Val Ala Gly Asn Leu Ser Ala Arg Gly Asp Gly
           835                 840                 845
   Ala Ala Gly Phe Thr Leu Pro Gly Phe Arg Phe Leu Pro Pro Pro His
       850                 855                 860
   Pro Pro Ser Ala Asn Leu Val Pro Leu Lys Pro Glu Glu His Ala Ile
   865                 870                 875                 880
   Lys Phe Glu Val Cys Val Asp Ala Leu Pro Ala Ile Asp Gly Leu Asp
   885 890 895
   Ser Thr Thr Cys Val His Gly Ala Ser Phe Ser Asp Ser Glu Asp Glu
   900 905 910
   Ser Cys Val Pro Leu Leu Arg Lys Glu Ser Ile Gln Leu Arg Asp Leu
   915 920 925
   Asp Ser Ala Leu Leu Ala Glu Val Lys Asp Val Leu Ile Pro His Glu
   930 935 940
   Arg Val Val Thr His Ser Asp Arg Val Ile Gly Lys Gly His Phe Gly
   945 950 955 960
   Val Val Tyr His Gly Glu Tyr Ile Asp Gln Ala Gln Asn Arg Ile Gln
   965 970 975
   Cys Ala Ile Lys Ser Leu Ser Arg Ile Thr Glu Met Gln Gln Val Glu
   980 985 990
   Ala Phe Leu Arg Glu Gly Leu Leu Met Arg Gly Leu Asn His Pro Asn
   995 1000 1005
   Val Leu Ala Leu Ile Gly Ile Met Leu Pro Pro Glu Gly Leu Pro
   1010 1015 1020
   His Val Leu Leu Pro Tyr Met Cys His Gly Asp Leu Leu Gln Phe
   1025 1030 1035
   Ile Arg Ser Pro Gln Arg Asn Pro Thr Val Lys Asp Leu Ile Ser
   1040 1045 1050
   Phe Gly Leu Gln Val Ala Arg Gly Met Glu Tyr Leu Ala Glu Gln
   1055 1060 1065
   Lys Phe Val His Arg Asp Leu Ala Ala Arg Asn Cys Met Leu Asp
   1070 1075 1080
   Glu Ser Phe Thr Val Lys Val Ala Asp Phe Gly Leu Ala Arg Asp
   1085 1090 1095
   Ile Leu Asp Arg Glu Tyr Tyr Ser Val Gln Gln His Arg His Ala
   1100 1105 1110
   Arg Leu Pro Val Lys Trp Met Ala Leu Glu Ser Leu Gln Thr Tyr
   1115 1120 1125
   Arg Phe  Thr Thr Lys Ser Asp  Val Val Pro Ser Asp  Ala Ala Met
       1130                 1135                 1140
   Leu Gly  Gly Arg Pro Ser Ser  Ala Thr His Leu Gln  Ser Thr Ser
       1145                 1150                 1155
   Gly Gly  Gly Gly Ala Asp Ser  Val Cys Thr Ala Trp  Gly Pro Leu
       1160                 1165                 1170
   Cys Ala  Ala Ala Ser Asn Leu  His Glu Leu Glu Leu  Thr Pro Arg
       1175                 1180                 1185
   Leu Pro  Leu Gly His Ala Arg  Pro Glu Gln Trp Pro  Ser Thr Leu
       1190                 1195                 1200
   Phe Leu  Pro Phe Asn Phe Gln  Arg Gln
       1205                 1210

## Description

### FIELD OF THE INVENTION

The invention relates to the nucleic acid and polypeptide sequences of a novel human Ron-related gene variant, preparation process thereof, and uses of the same in diagnosing cancers, in particular, breast carcinoma, breast adenocarcinoma, cervix epidermoid carcinoma, cervix epitheloid carcinoma, colon adenocarcinoma, urinary bladder carcinoma, prostate carcinoma, esophagus epidermoid carcinoma and esophagus carcinoma.

### BACKGROUND OF THE INVENTION

Cancer is one of the major causes of death in the world. According to the report from the WHO Fact Sheet-Cancer (2003), cancer accounts for 7.1 million (12.6% of the global total) deaths annually and new cancer cases are expected to be increased from 10 million people in 2000 to 15 million people by 2020. In recent years, much progress has been made toward understanding the molecular and cellular biology of cancers. Many important contributions have been made by the identification of several key genetic factors associated with cancers. However, the treatments of cancers still mainly depend on surgery, chemotherapy and radiotherapy because the molecular mechanisms underlying the pathogenesis of cancers remain largely unclear.

It is interesting to note that receptor tyrosine kinases are a family of proteins reported to be involved in many fundamental cellular processes such as cell cycle, signalling transduction, proliferation, differentiation, adhesion, migration, and apoptosis. An important role of the receptor tyrosine kinases in the development of cancers has been reported previously. Therefore, future strategies for the prevention and treatment of cancers may be focused on the elucidation of the receptor tyrosine kinases which are involved in the pathogenesis of cancers. Ron, a member of the receptor tyrosine kinase family located on human chromosome 3p21 (a region frequently deleted in small cell lung carcinoma), was isolated and identified through the screening of cDNA libraries. Previous studies have indicated that Ron is involved in many genetic mechanisms of the tumor cells (Wang et al., (2003) Carcinogenesis 24:1291-300; Angeloni et al., (2004) J Biol Chem. 279:3726-32; Peace et al., (2005) Cancer Res. 65:1285-93; Camp et al., (2005) Ann Surg Oncol. 12:273-81), and that Ron is a potential target for understanding the pathogenesis of cancers. In addition, the association between gene variants and diseases has been reported previously. Therefore, the discovery of gene variants of Ron may be important for the diagnostic markers of cancers.

### SUMMARY OF THE INVENTION

The present invention provides one Ron-related gene variant present in human breast carcinoma, breast adenocarcinoma, cervix epidermoid carcinoma, cervix epitheloid carcinoma, colon adenocarcinoma, urinary bladder carcinoma, prostate carcinoma, esophagus epidermoid carcinoma and esophagus carcinoma. The nucleotide sequence of the gene variant and polypeptide sequence encoded thereby can be used for the diagnosis of any diseases associated with this gene variant or cancers, in particular, breast carcinoma, breast adenocarcinoma, cervix epidermoid carcinoma, cervix epitheloid carcinoma, colon adenocarcinoma, urinary bladder carcinoma, prostate carcinoma, esophagus epidermoid carcinoma and esophagus carcinoma.

The invention further provides an expression vector and host cell for expressing the variant.

The invention further provides a method for producing the variant.

The invention further provides an antibody that specifically binds to the variant. For example, by selecting a sequence unique to one of the variants, e.g., a peptide fragment that spans one the deletion junctions, an antibody may be generated that binds to one of the variants and not to RON. Preferably, such peptide antigens are at least 17 amino acid long, but in some cases smaller peptides such as 10mers, 12mers, or 15mers may suffice. Longer peptides, such as 20mers, 50 mers, hundred-mers (and those therebetween) and even up to full length proteins may be used.

The invention also provides methods for detecting the presence of the variant in a mammal.

### BRIEF DESCRIPTION OF THE DRAWINGS

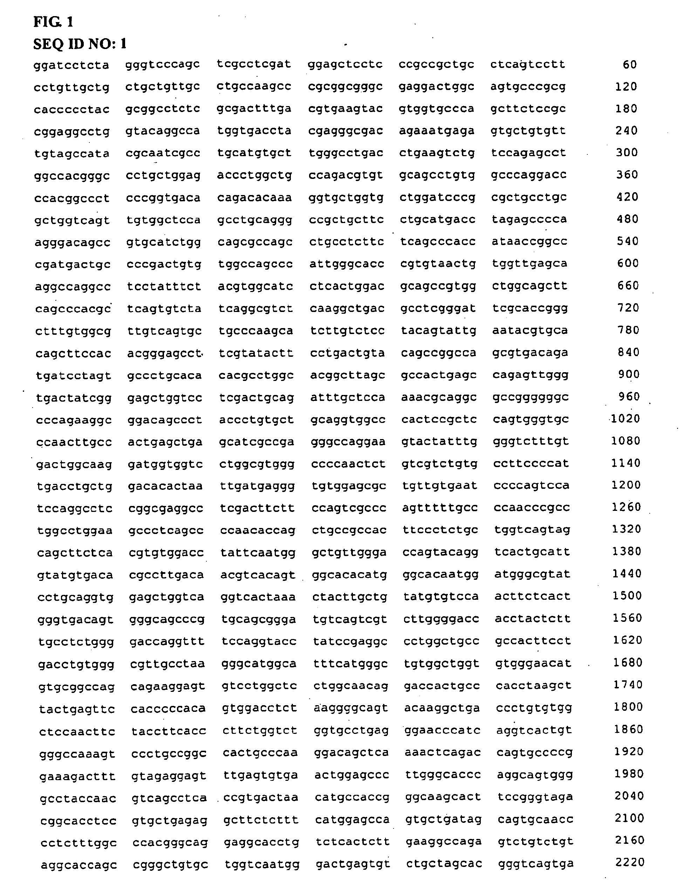

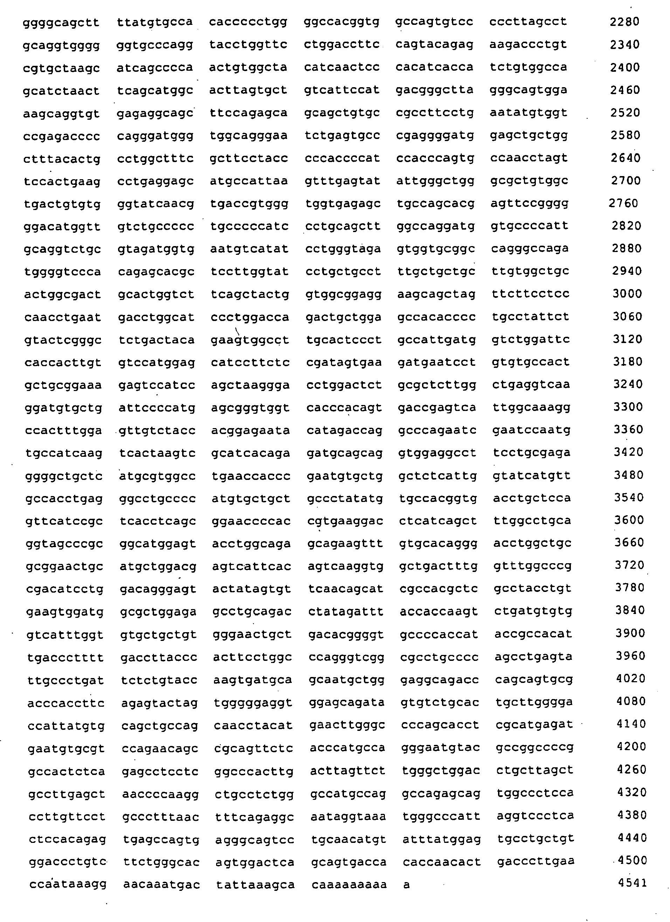

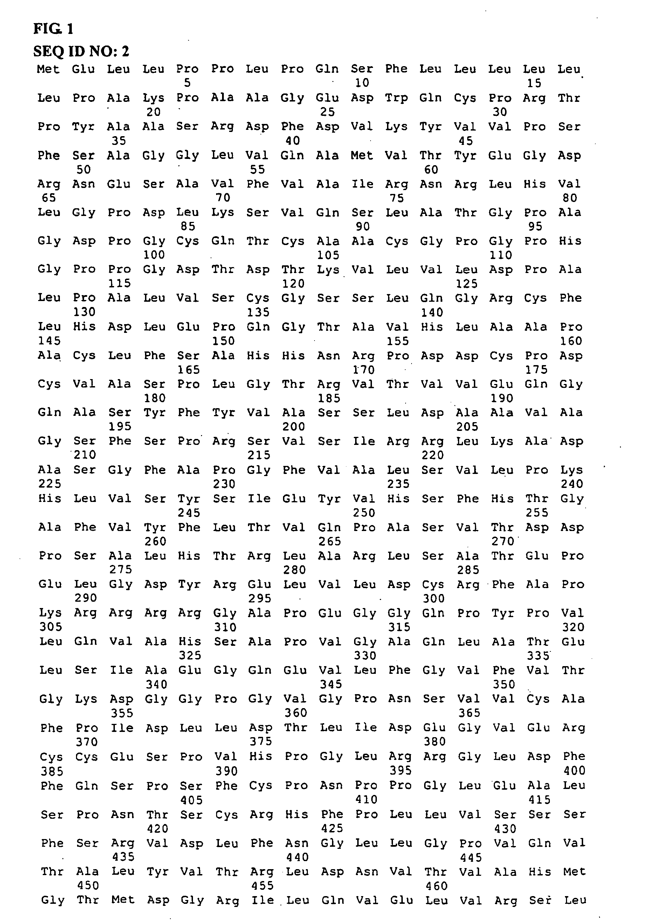

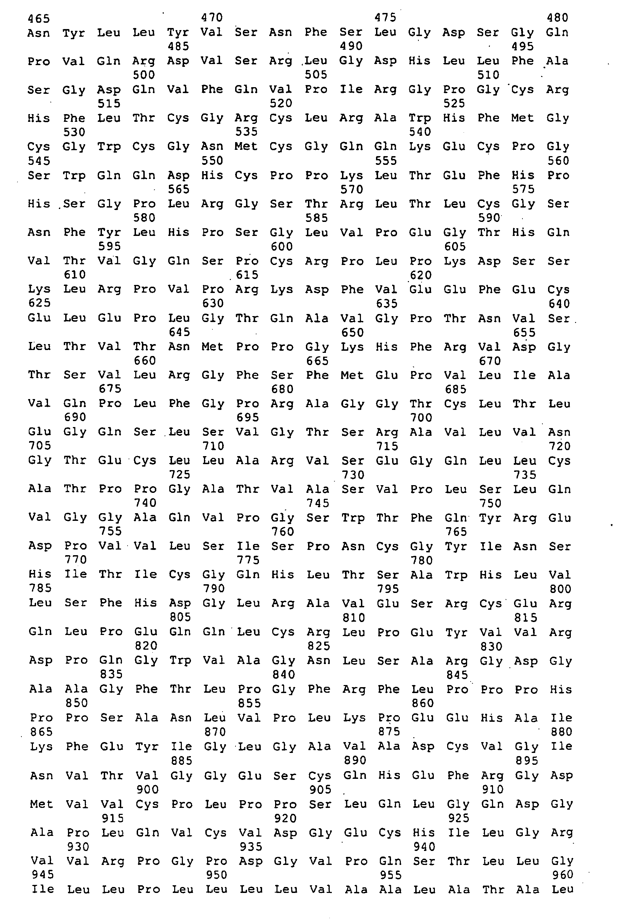

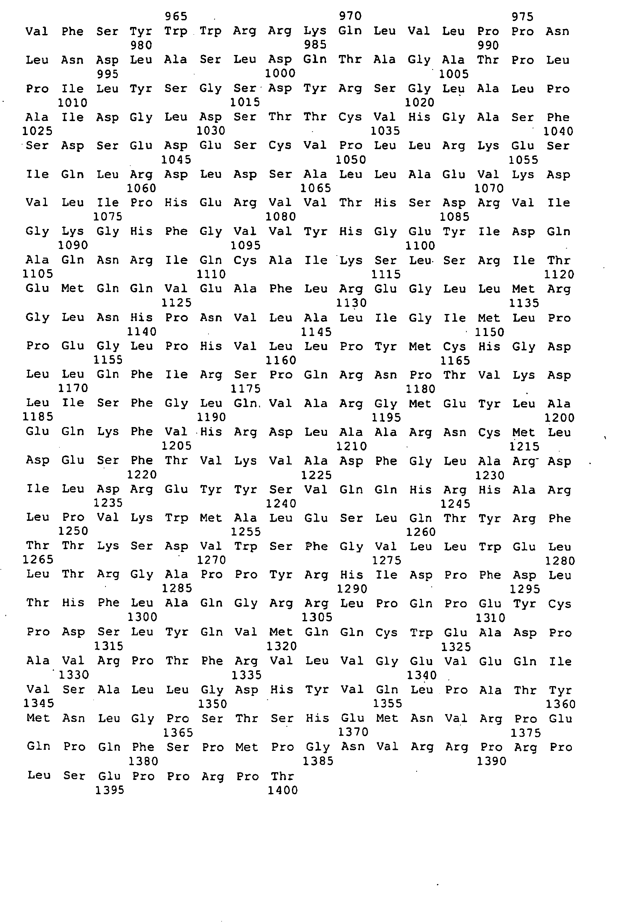

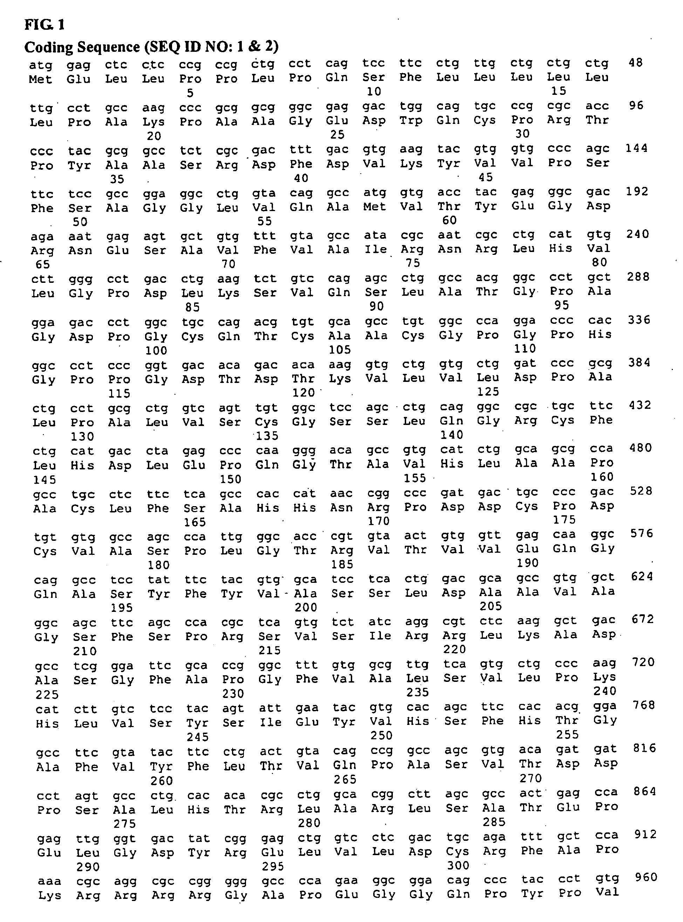

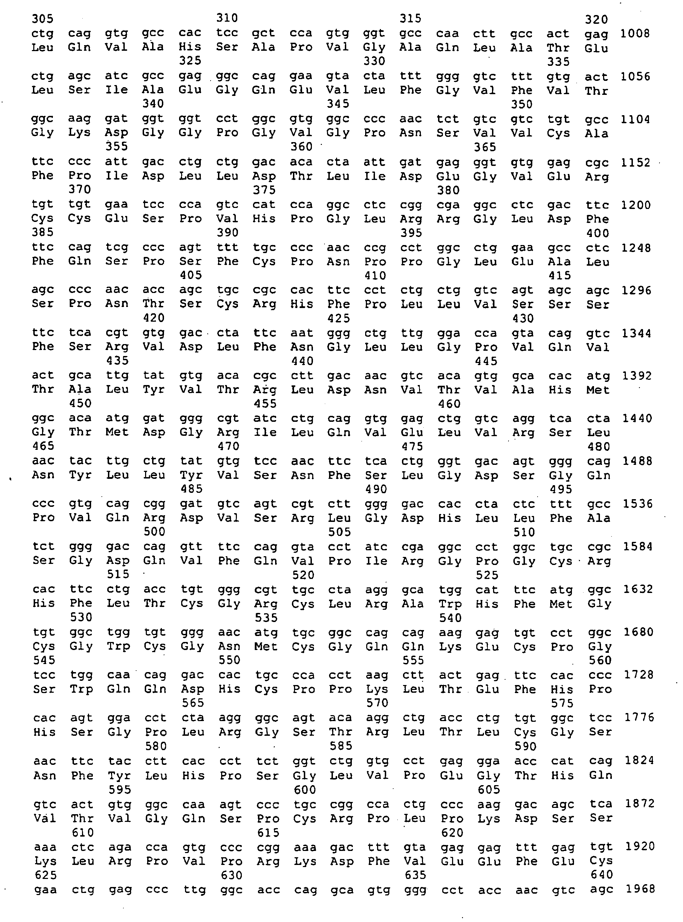

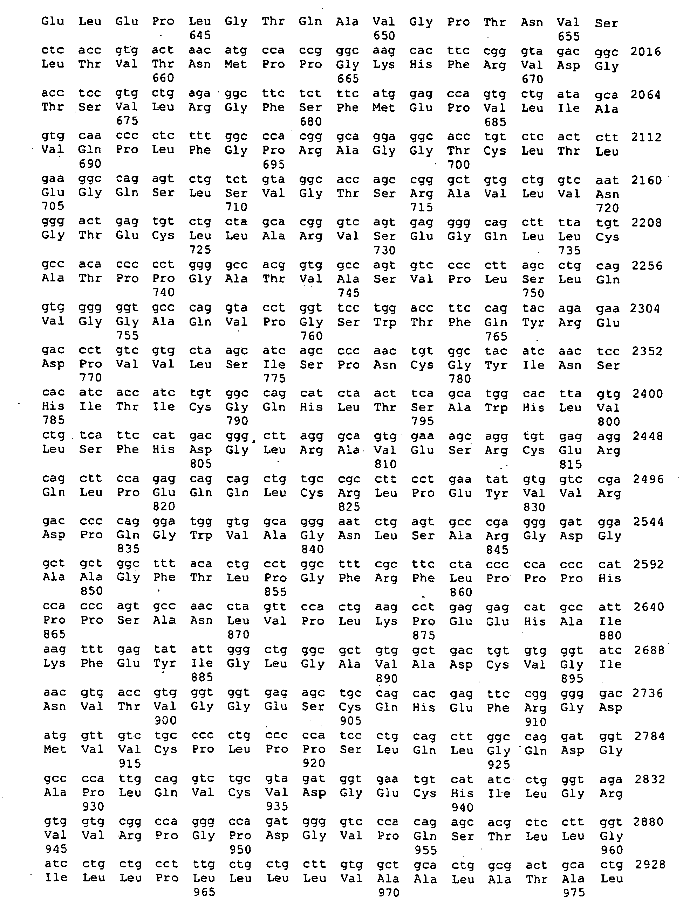

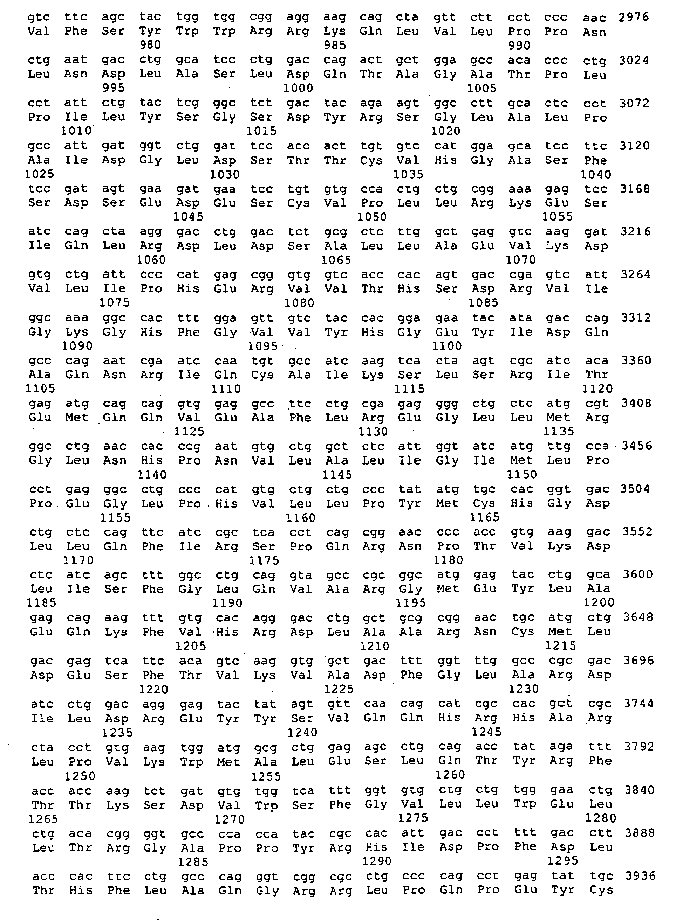

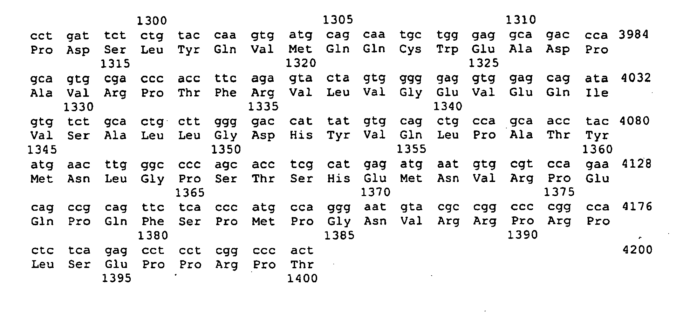

FIG. 1 shows the nucleic acid sequence (SEQ ID NO:1), amino acid sequence (SEQ ID NO:2) and nucleic acid with corresponding amino acid sequence (SEQ ID NO: 1 & 2) of Ron.

FIG. 2 shows the nucleic acid sequence (SEQ ID NO:3), amino acid sequence (SEQ ID NO:4) and nucleic acid with corresponding amino acid sequence (SEQ ID NO: 3 & 4) of Ron-V1.

FIG. 3 shows the nucleic acid sequence (SEQ ID NO:5), amino acid sequence (SEQ ID NO:6) and nucleic acid with corresponding amino acid sequence (SEQ ID NO: 5 & 6) of Ron-V2.

FIG. 4 shows the nucleic acid sequence (SEQ ID NO:7), amino acid sequence (SEQ ID NO:8) and nucleic acid with corresponding amino acid sequence (SEQ ID NO: 7 & 8) of Ron-V3.

FIG. 5 shows the nucleotide sequence alignment between the human Ron gene (SEQ ID NO: 1) and its related gene variants (Ron-V1, Ron-V2, and Ron-V3, SEQ ID NO: ID 3, 5, 7).

FIG. 6 shows the amino acid sequence alignment between the human Ron protein (SEQ ID NO: 2) and its related gene variants (Ron-V1, Ron-V2, and Ron-V3) (SEQ ID NO: 4, 6, 8).

FIG. 7 shows the expression pattern of Ron gene variants in human cell lines.

### DETAILED DESCRIPTION OF THE INVENTION

All technical and scientific terms used herein, unless otherwise defined, have the same meanings as commonly understood by persons skilled in the art.

The term "antibody" used herein denotes intact molecules (a polypeptide or group of polypeptides) as well as fragments thereof, such as Fab, R(ab')2, and Fv fragments, which are capable of binding epitopes. Antibodies are produced by specialized B cells after stimulation by an antigen. Structurally, antibody consists of four subunits including two heavy chains and two light chains. The internal surface shape and charge distribution of the antibody binding domain is complementary to the features of an antigen. Thus, antibody can specifically act against the antigen in an immune response.

The term "base pair (bp)" used herein denotes nucleotides composed of a purine on one strand of DNA which can be hydrogen bonded to a pyrimidine on the other strand. Thymine (or uracil) and adenine residues are linked by two hydrogen bonds. Cytosine and guanine residues are linked by three hydrogen bonds.

The term "Basic Local Alignment Search Tool ("BLAST") (BLAST; Altschul et al., (1997) Nucleic Acids Res. 25: 3389-3402) used herein denotes programs for evaluation of homologies between a query sequence (amino or nucleic acid) and a test sequence. Specific BLAST programs are described as follows:

(1) BLASTN compares a nucleotide query sequence against a nucleotide sequence database;

(2) BLASTP compares an amino acid query sequence against a protein sequence database;

(3) BLASTX compares the six-frame conceptual translation products of a query nucleotide sequence against a protein sequence database;

(4) TBLASTN compares a query protein sequence against a nucleotide sequence database translated in all six reading frames; and

(5) TBLASTX compares the six-frame translations of a nucleotide query sequence against the six-frame translations of a nucleotide sequence database.

The term "cDNA" used herein denotes nucleic acids synthesized from a mRNA template using reverse transcriptase.

The term "cDNA library" used herein denotes a library composed of complementary DNAs which are reverse-transcribed from mRNAs.

The term "complement" used herein denotes a polynucleotide sequence capable of forming base pairing with another polynucleotide sequence. For example, the sequence 5'-ATGGACTTACT-3' binds to the complementary sequence 5'-AGTAAGTCCAT-3'.

The term "deletion" used herein denotes a removal of a portion of one or more amino acid residues/nucleotides from a gene.

The term "expressed sequence tags (ESTs)" used herein denotes short (200 to 500 base pairs) nucleotide sequence that derives from either 5' or 3' end of a cDNA.

The term "expression vector" used herein denotes nucleic acid constructs which contain a cloning site for introducing the DNA into vector, one or more selectable markers for selecting vectors containing the DNA, an origin of replication for replicating the vector whenever the host cell divides, a terminator sequence, a polyadenylation signal, and a suitable control sequence which can effectively express the DNA in a suitable host. The suitable control sequence may include promoter, enhancer and other regulatory sequences necessary for directing polymerases to transcribe the DNA.

The term "host cell" used herein denotes a cell which is used to receive, maintain, and allow the reproduction of an expression vector comprising DNA. Host cells are transformed or transfected with suitable vectors constructed using recombinant DNA methods. The recombinant DNA introduced with the vector is replicated whenever the cell divides.

The term "insertion" or "addition" used herein denotes the addition of a portion of one or more amino acid residues/nucleotides to a gene.

The term "in silico" used herein denotes a process of using computational methods (e.g., BLAST) to analyze DNA sequences.

The term "polymerase chain reaction (PCR)" used herein denotes a method which increases the copy number of a nucleic acid sequence using a DNA polymerase and a set of primers (about 20bp oligonucleotides complementary to each strand of DNA) under suitable conditions (successive rounds of primer annealing, strand elongation, and dissociation).

The term "polynucleotide" or "nucleic acid sequence" used herein denotes a sequence of nucleotide (guanine, cytosine, thymine or adenine) in a specific order that can be a natural or synthesized fragment of DNA or RNA. It may be single-stranded or double-stranded.

The term "protein" or "polypeptide" used herein denotes a sequence of amino acids in a specific order that can be encoded by a gene or by a recombinant DNA. It can also be chemically synthesized.

The term "RNA interference (RNAi)" used herein denotes an introduction of homologous double stranded RNA (dsRNA) into a cell to specifically inhibit the expression of a gene.

The term "reverse transcriptase-polymerase chain reaction (RT-PCR)" used herein denotes a process which transcribes mRNA to complementary DNA strand using reverse transcriptase followed by polymerase chain reaction to amplify the specific fragment of DNA sequences.

The term "transformation" used herein denotes a process describing the uptake, incorporation, and expression of exogenous DNA by prokaryotic host cells.

The term "transfection" used herein denotes a process describing the uptake, incorporation, and expression of exogenous DNA by eukaryotic host cells.

The term "variant" used herein denotes a fragment of sequence (nucleotide or amino acid) inserted or deleted by one or more nucleotides/amino acids.

According to the present invention, the polypeptides of one novel human Ron-related gene variant and the nucleic acid sequences encoding the same are provided.

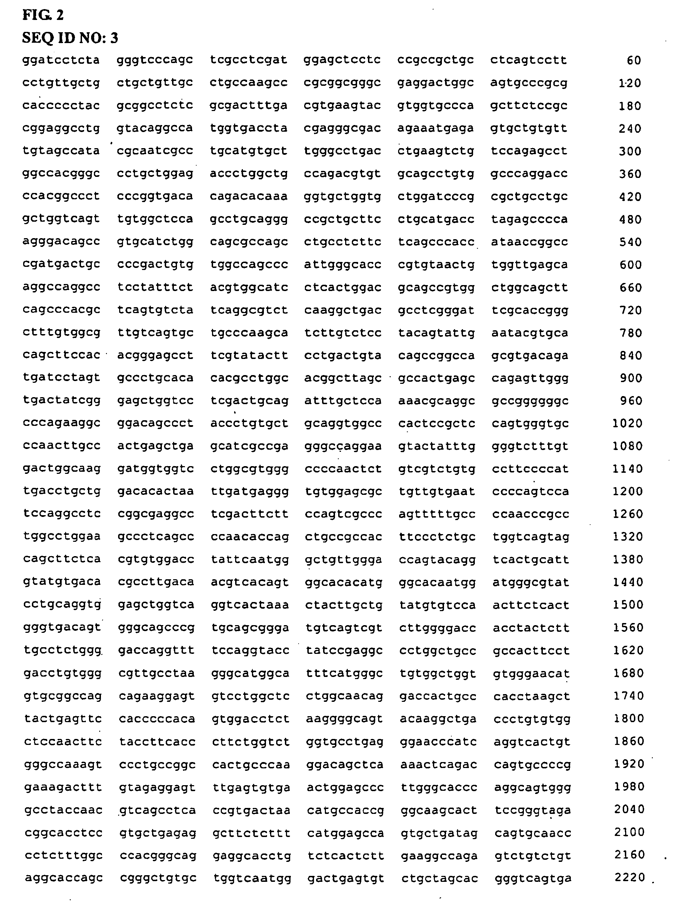

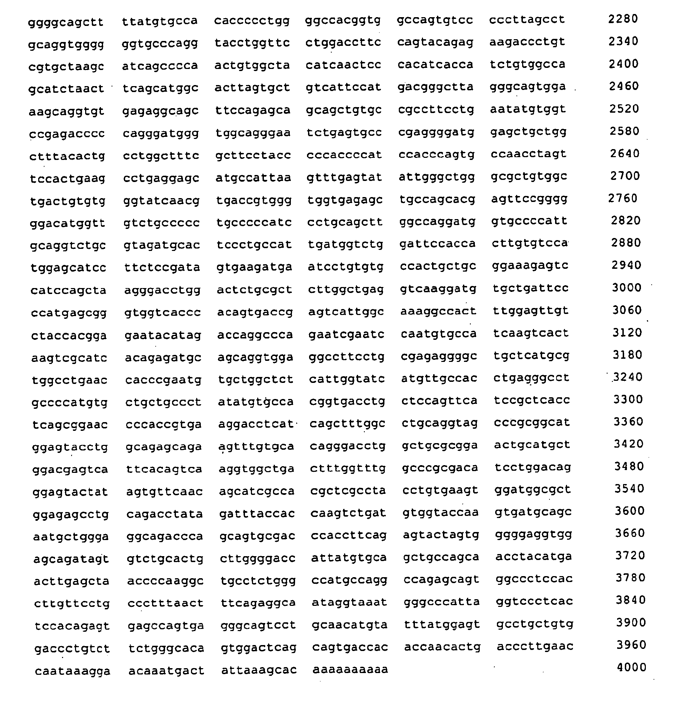

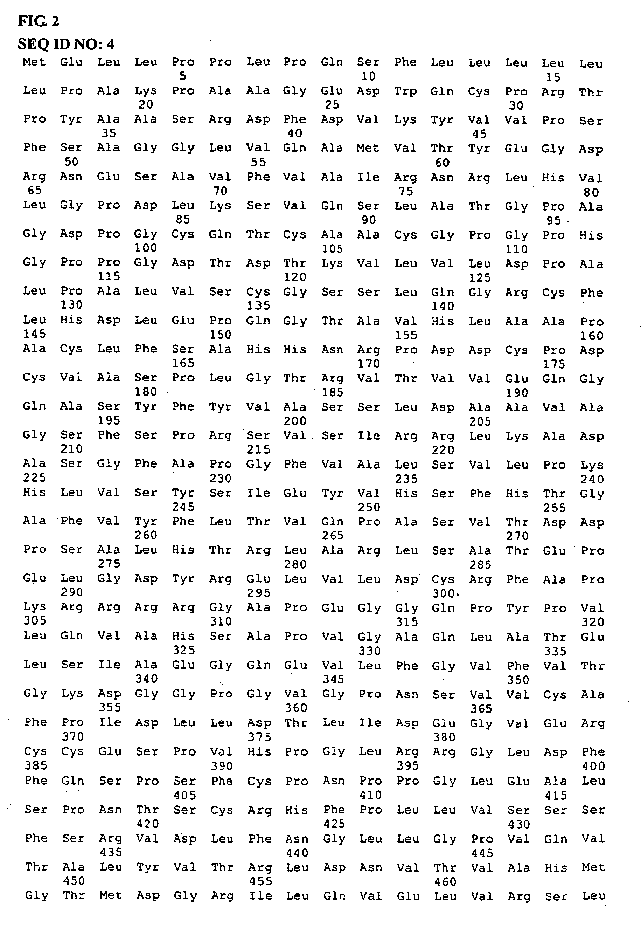

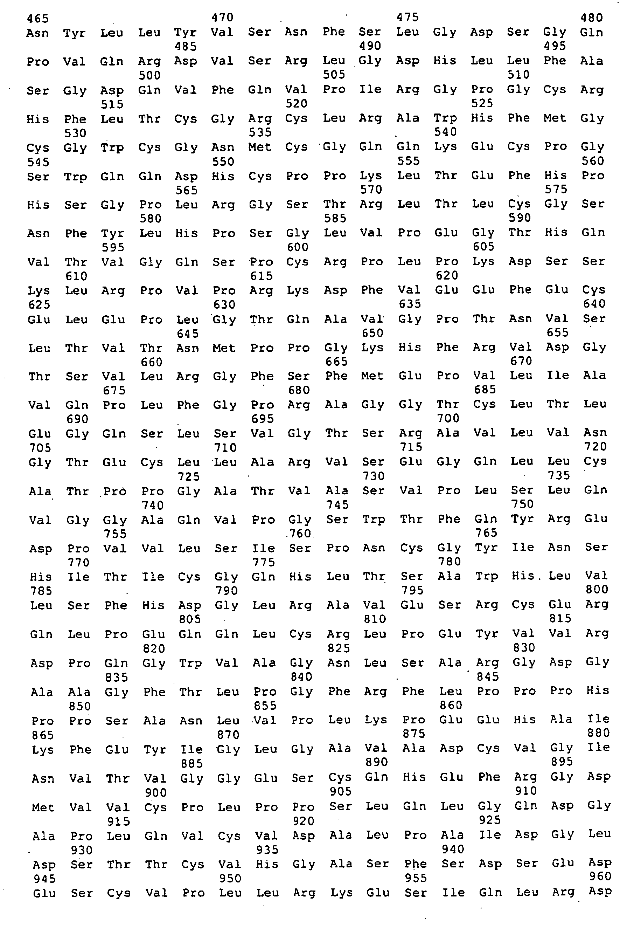

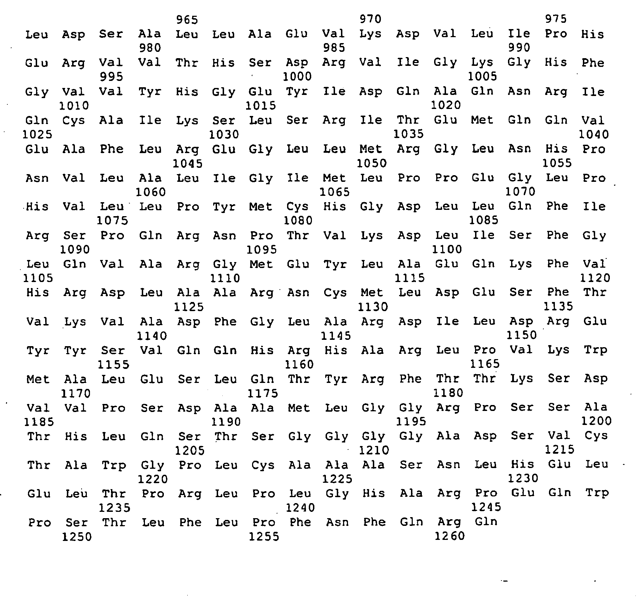

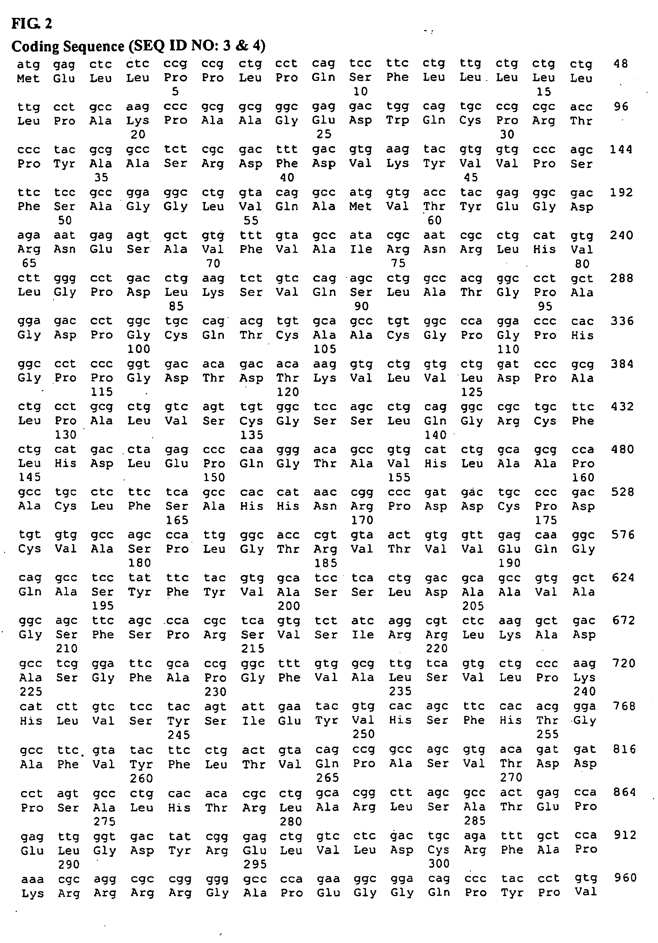

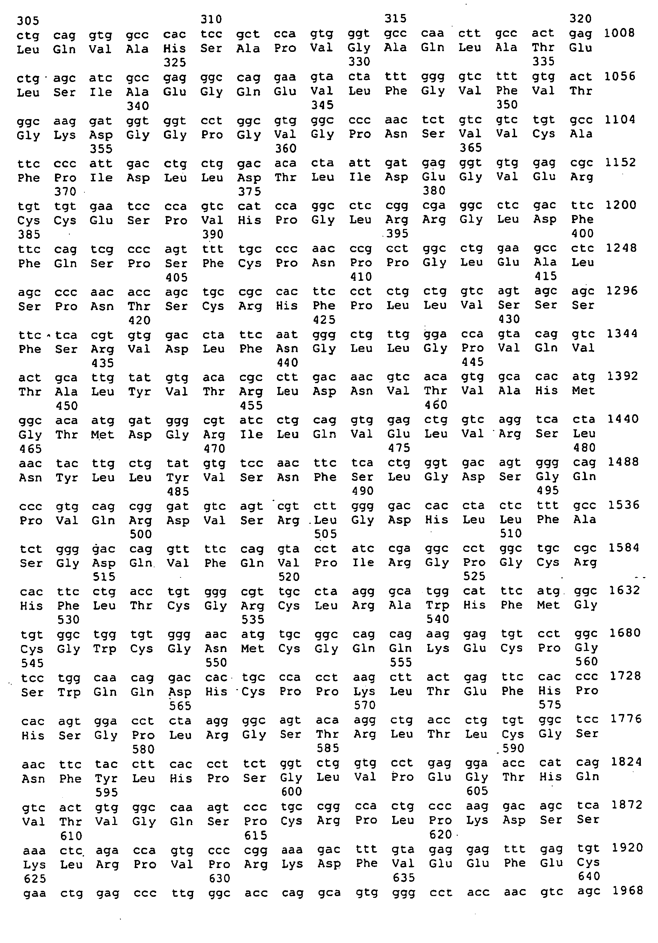

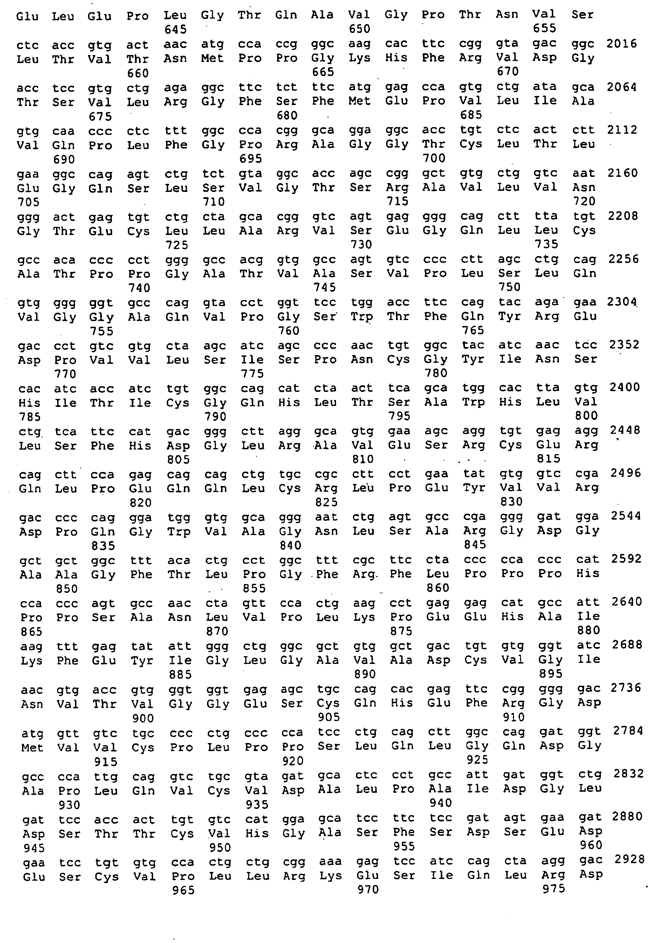

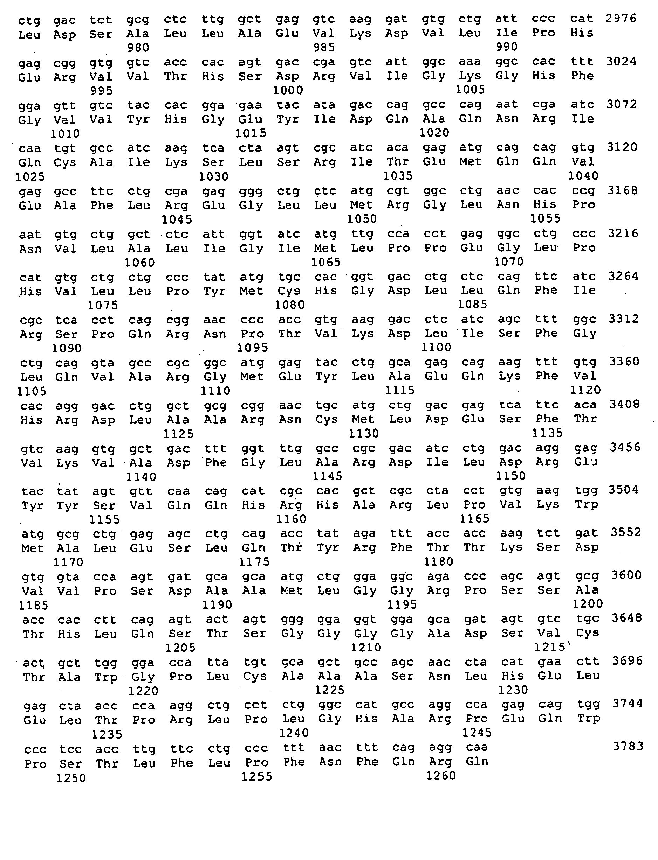

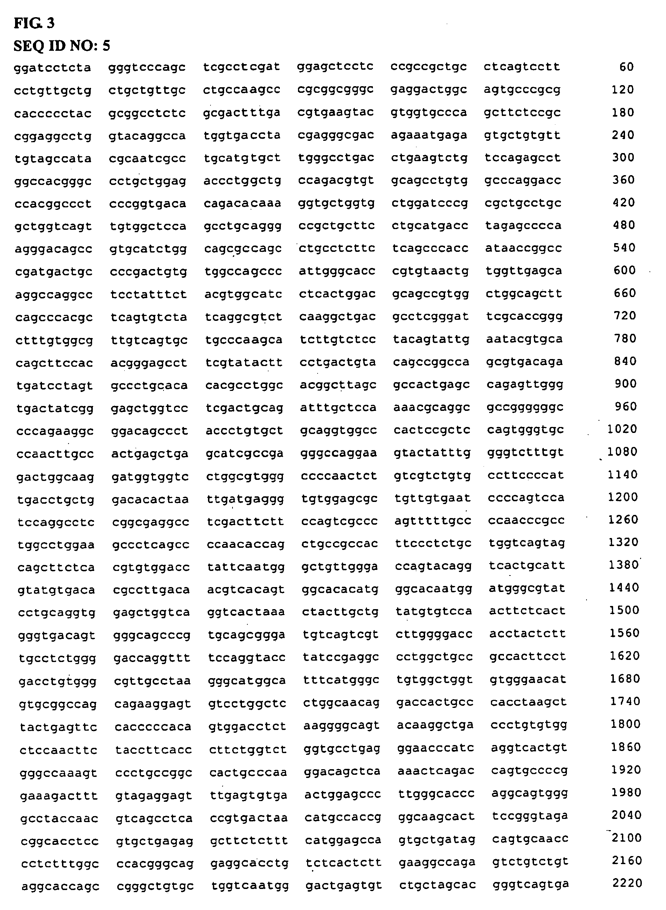

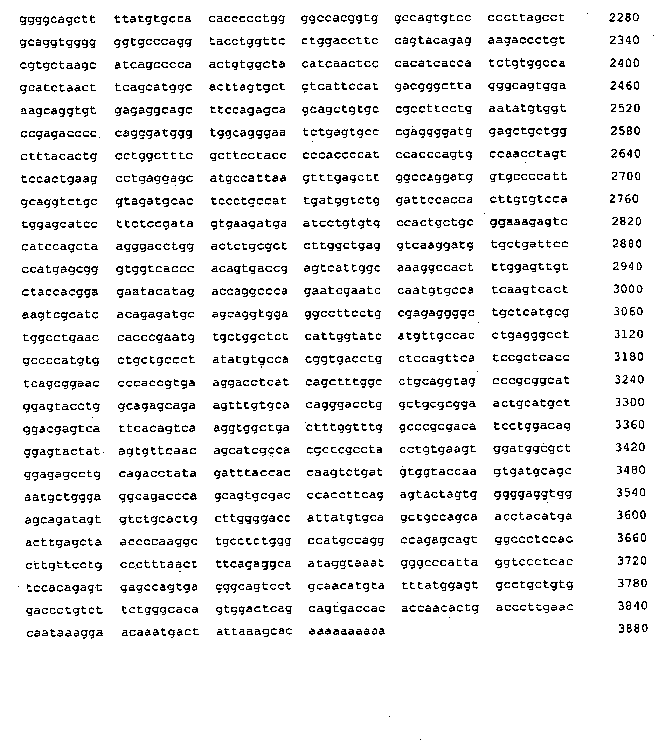

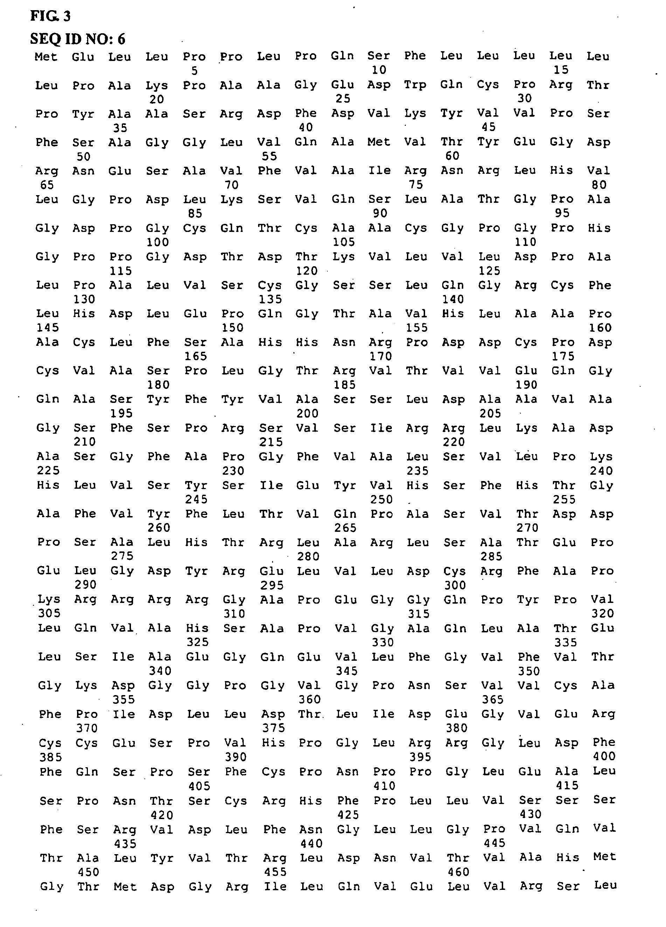

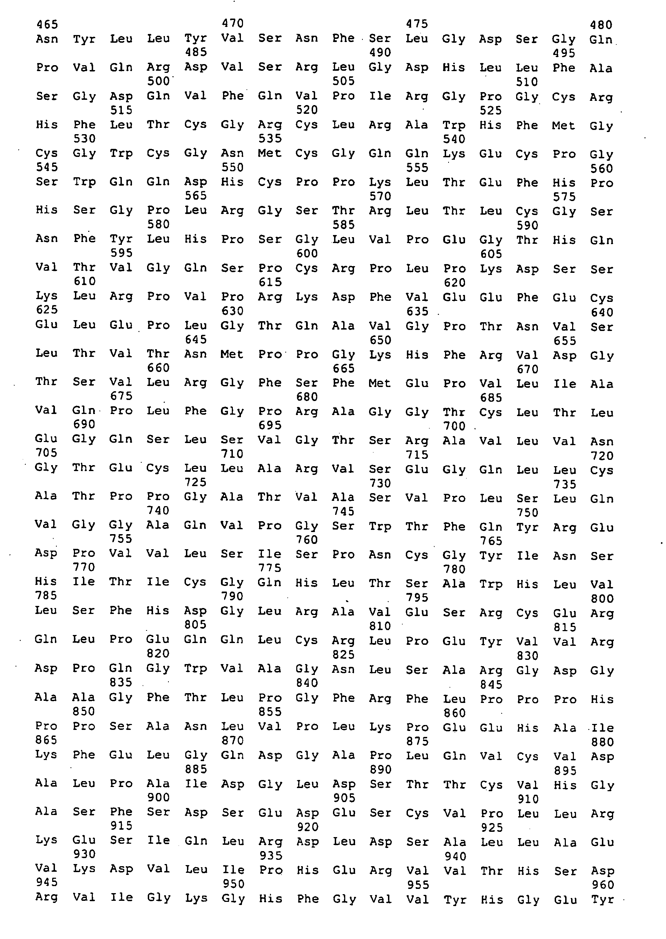

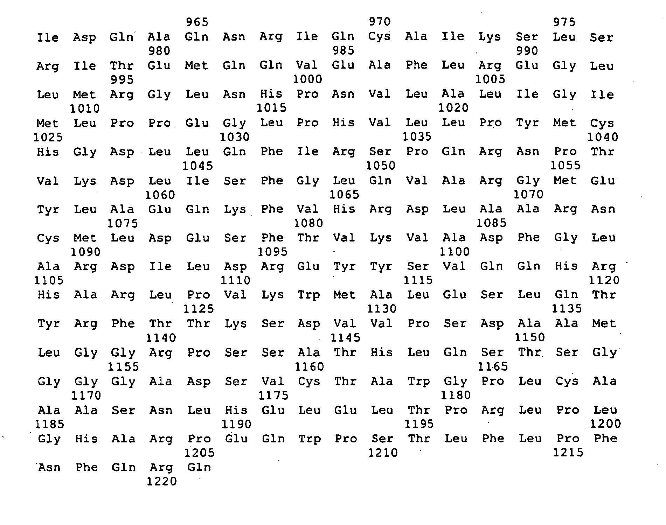

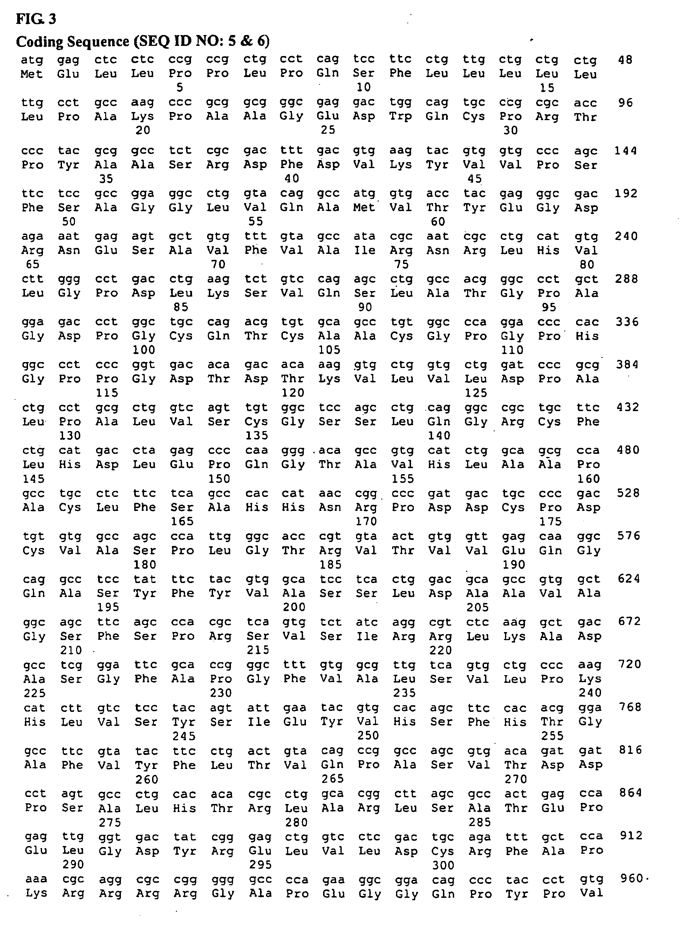

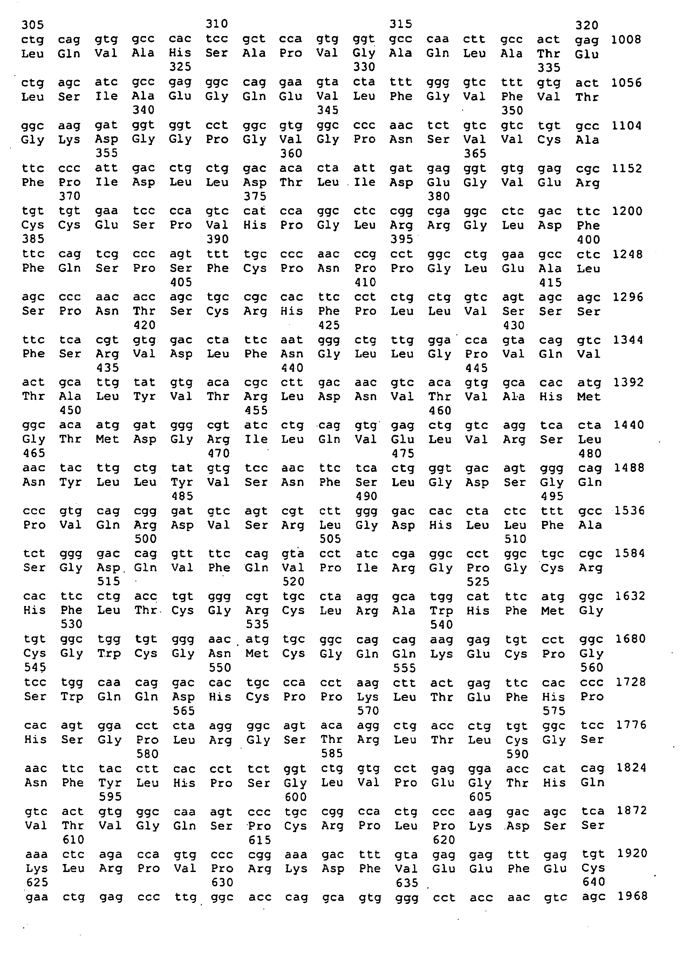

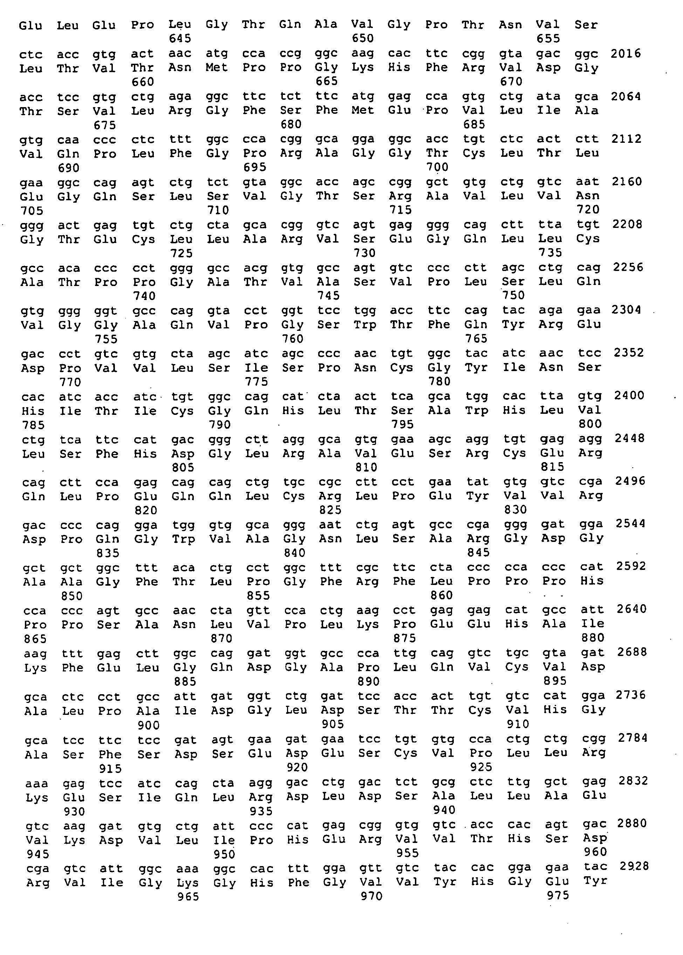

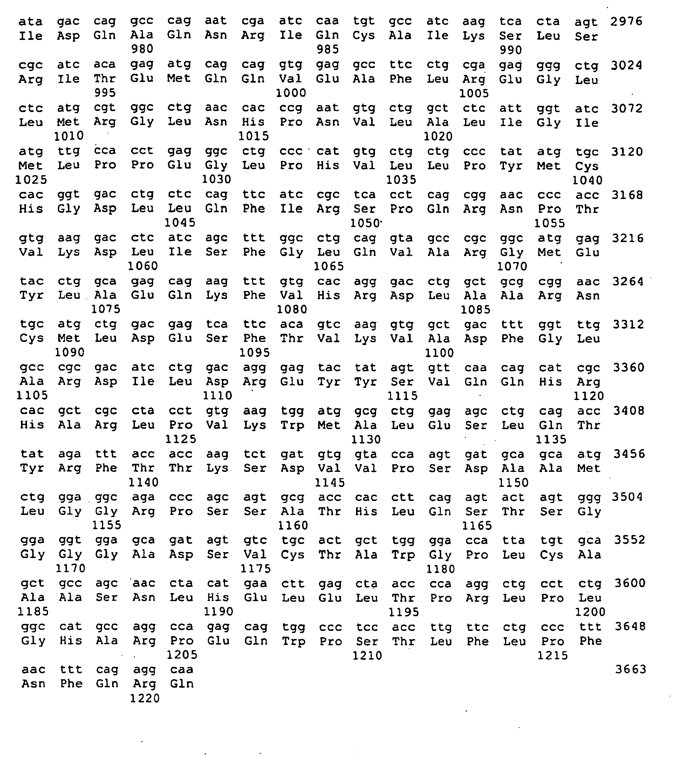

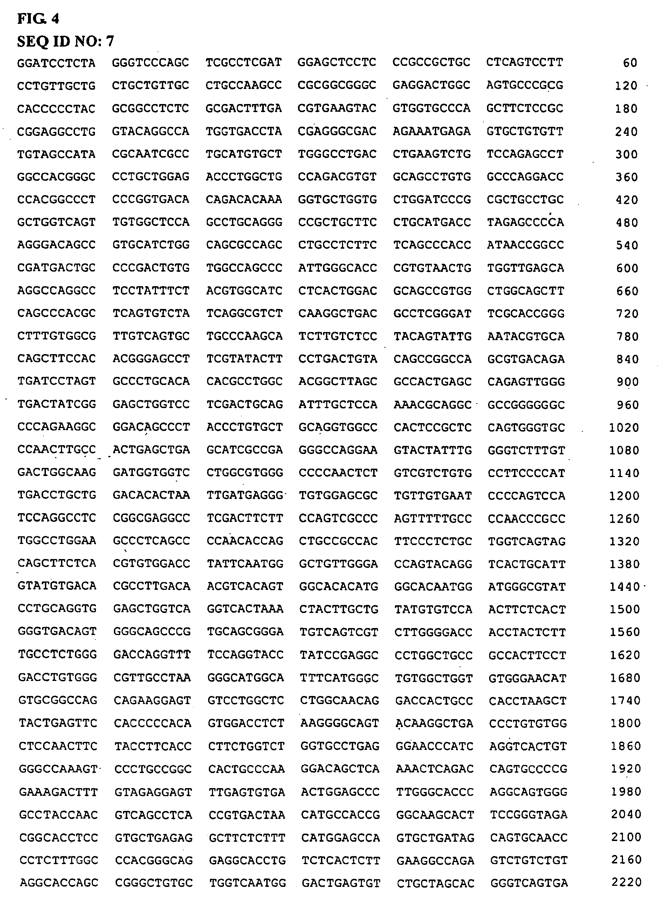

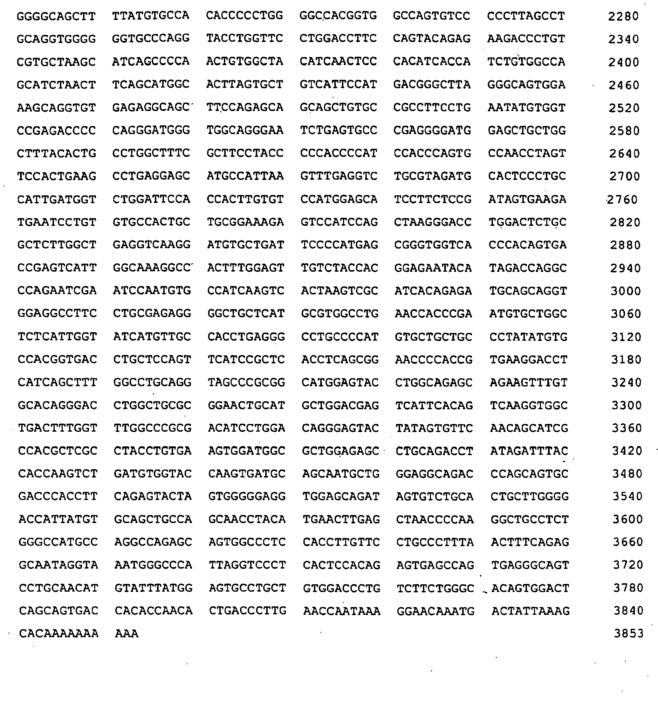

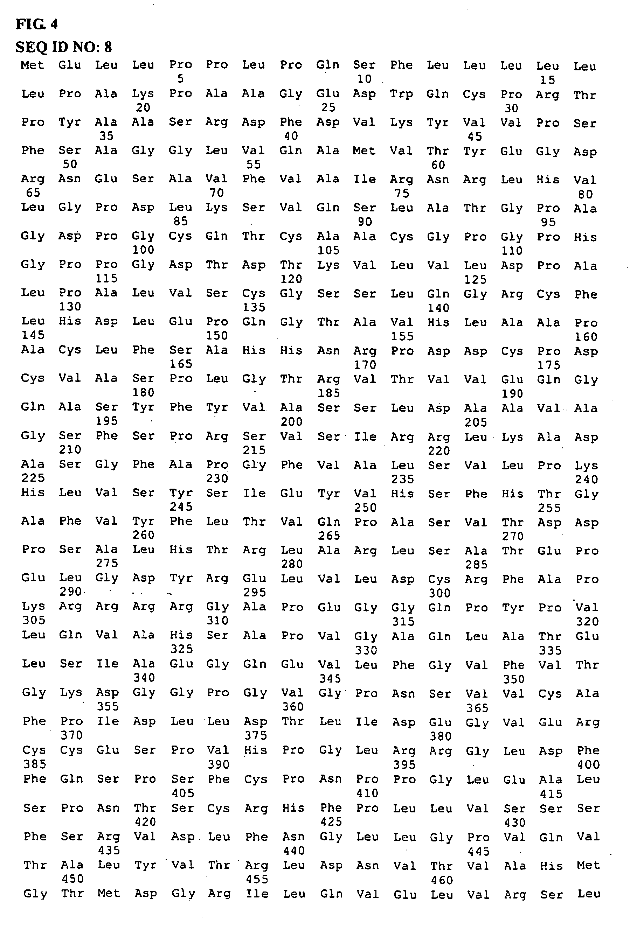

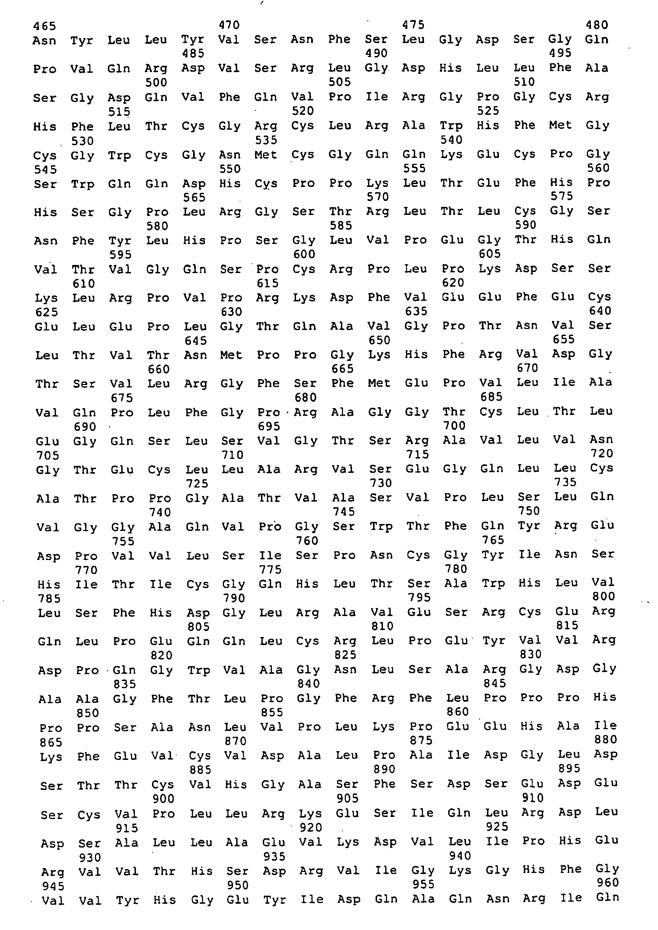

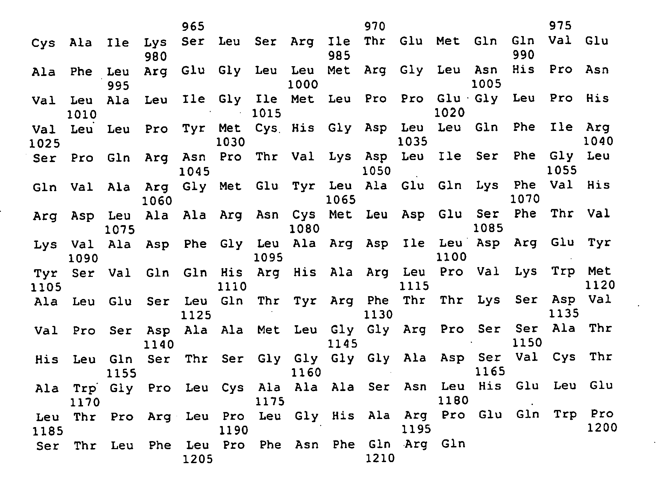

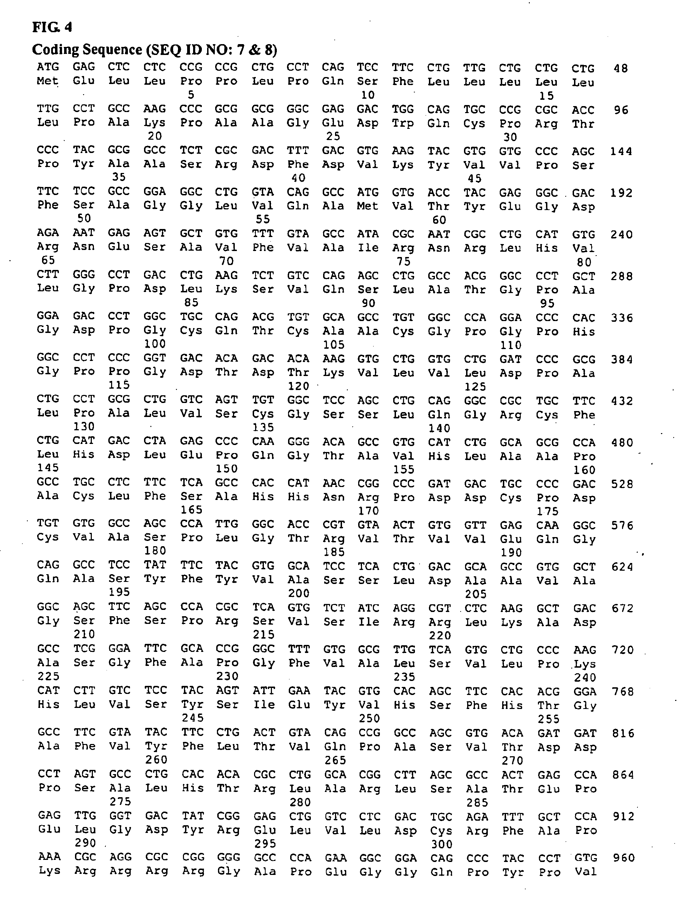

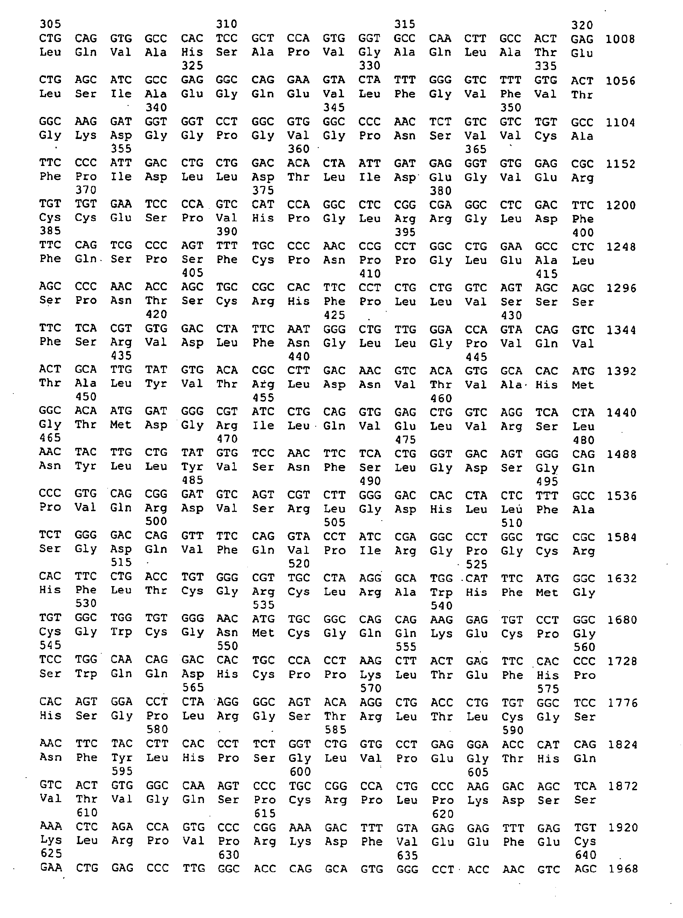

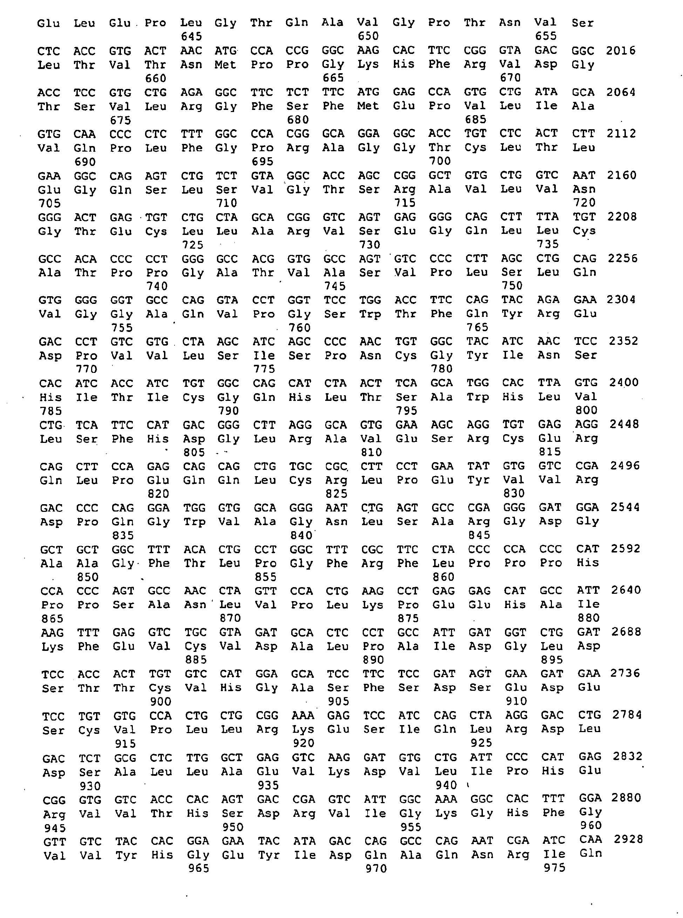

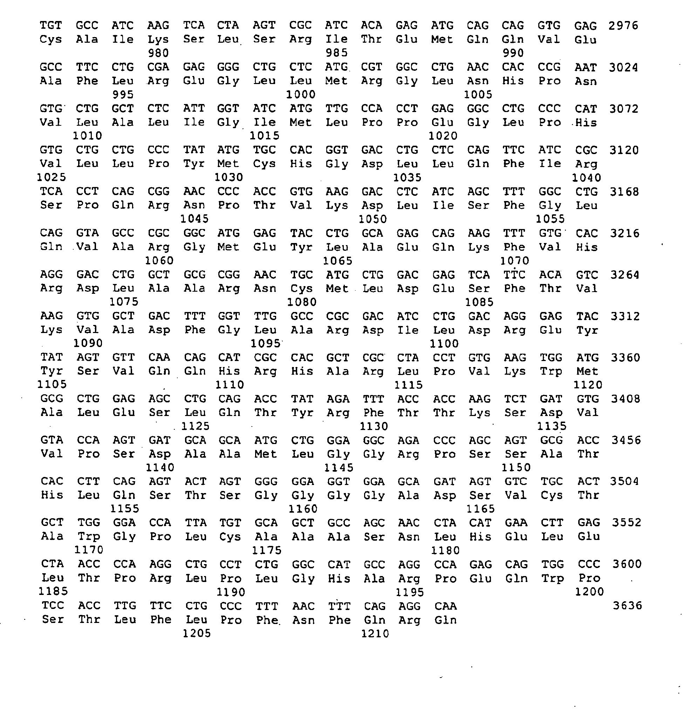

According to the present invention, human Ron cDNA sequence was used to query the human EST databases using BLAST program to search for Ron-related gene variants. Three human cDNA partial sequences (i.e., EST) showing similar to Ron was identified from ESTs deposited in a cDNA database constructed using a SW620 (colon adenocarcinoma) cell line. The cDNA clones, named Ron-V1 (Ron variant 1), Ron-V2 (Ron variant 2), and Ron-V3 (Ron variant 3), were then isolated from the SW620 cDNA library and sequenced. FIGs. 2-4 show the nucleic acid sequences (SEQ ID NOs: 3, 5, & 7) of Ron-V1, Ron-V2, and Ron-V3 and its corresponding amino acid sequences (SEQ ID NOs: 4, 6, & 8) encoded thereby.

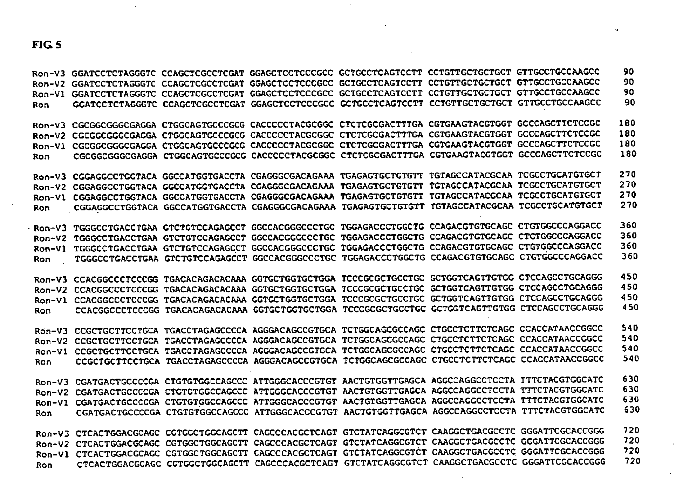

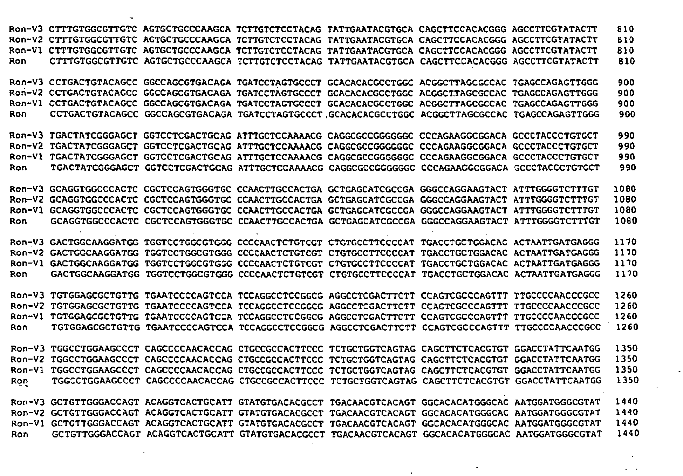

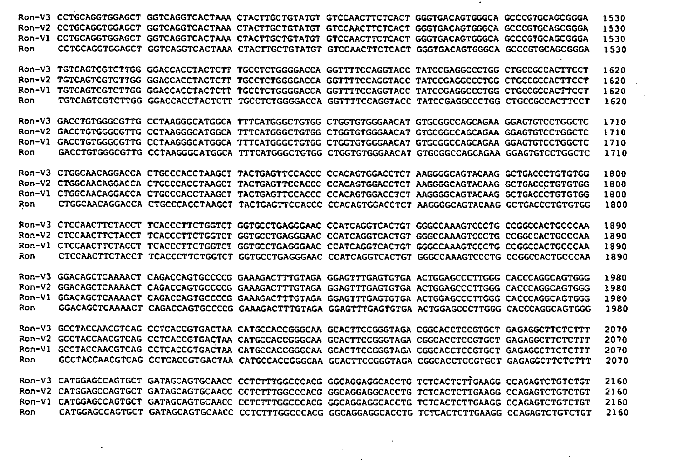

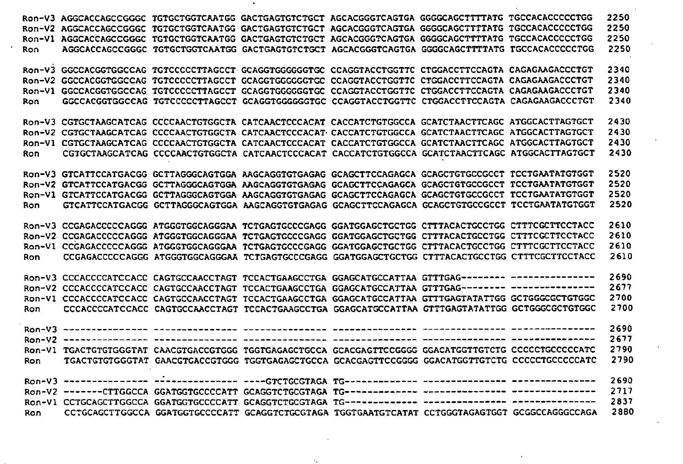

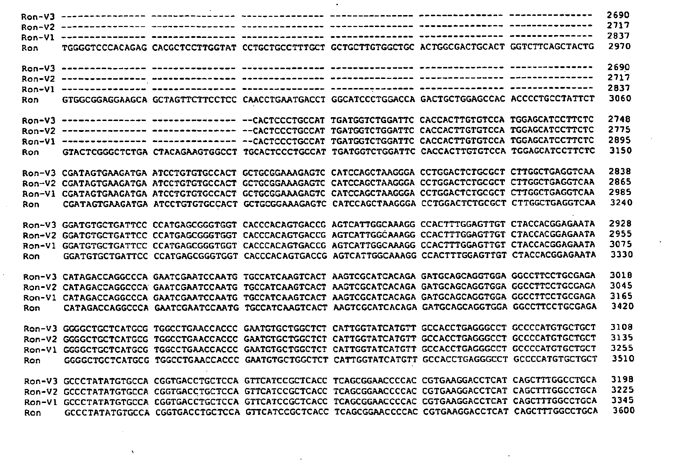

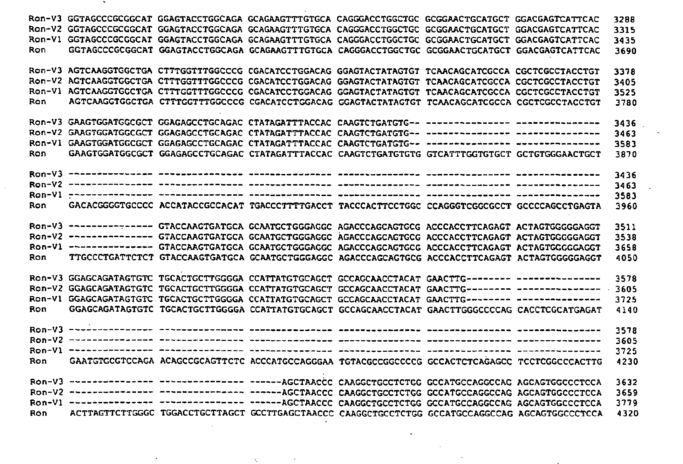

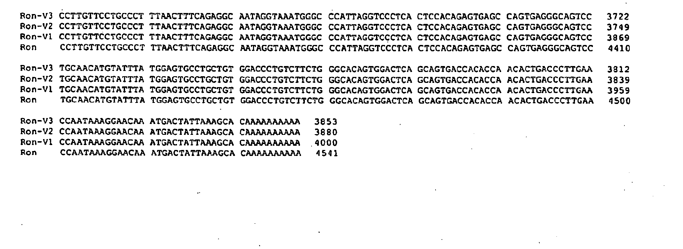

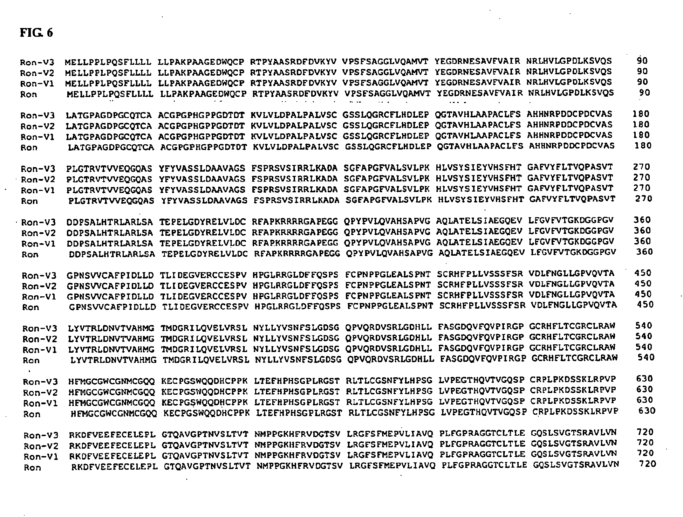

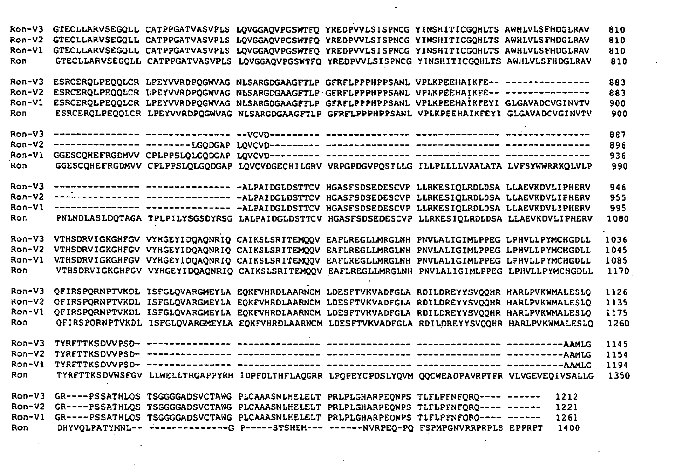

The full-length of the Ron-V1 cDNA is a 4000bp clone containing a 3783bp open reading frame (ORF) extending from 29bp to 3811bp, which corresponds to an encoded protein of 1261 amino acid residues with a predicted molecular mass of 136.1 kDa. The initiation ATG sequence of Ron-V1 is located at nucleotide 29-31bp. The full-length of the Ron-V2 cDNA is a 3880bp clone containing a 3663bp open reading frame (ORF) extending from 29bp to 3691bp, which corresponds to an encoded protein of 1221 amino acid residues with a predicted molecular mass of 132 kDa. The initiation ATG sequence of Ron-V2 is located at nucleotide 29-31bp. The full-length of the Ron-V3 cDNA is a 3853bp clone containing a 3636bp open reading frame (ORF) extending from 29bp to 3664bp, which corresponds to an encoded protein of 1212 amino acid residues with a predicted molecular mass of 131.1 kDa. The initiation ATG sequence of Ron-V3 is located at nucleotide 29-31bp. To determine the variations in sequence of Ron-V1, Ron-V2 and Ron-V3 cDNA clones, an alignment of Ron nucleotide/amino acid sequence with Ron-V1, Ron-V2 and Ron-V3 was performed (FIGs. 5 and 6). The results indicate that (1) Ron-V1: three major genetic deletions were found in the aligned nucleotide sequences showing that Ron-V1 has a 255bp, a 137bp and a 149bp deletions in the sequence of Ron from nucleotides 2838-3092, nucleotides 3839-3975 and nucleotides 4118-4266, respectively; (2) Ron-V2: four major genetic deletions were found in the aligned nucleotide sequences showing that Ron-V2 has a 120bp, a 255bp, a 137bp and a 149bp deletions in the sequence of Ron from nucleotides 2678-2797, nucleotides 2838-3092, nucleotides 3839-3975 and nucleotides 4118-4266, respectively; (3) Ron-V3: four major genetic deletions were found in the aligned nucleotide sequences showing that Ron-V3 has a 147bp, a 255bp, a 137bp and a 149bp deletions in the sequence of Ron from nucleotides 2678-2824, nucleotides 2838-3092, nucleotides 3839-3975 and nucleotides 4118-4266, respectively. Scanning Ron, Ron-V1, Ron-V2 and Ron-V3 amino acid sequences against protein profile databases including PROSITE (Motif Scan) indicated that Ron protein contains one Sema domain (31-522aa), one Protein_Kinase_ATP (1088-1114aa), one Protein_Kinase_TYR (1204-1216aa), and one Protein_Kinase_DOM (1082-1345aa). Ron-V1 protein contains one Sema domain (31-522aa), one Protein_Kinase_ATP (1003-1029aa), one Protein_Kinase_TYR (1119-1131aa), and one Protein_Kinase_DOM (997-1261aa). Ron-V2 protein contains one Sema domain (31-522aa), one Protein_Kinase_ATP (963-989aa), one Protein_Kinase_TYR (1079-1091aa), and one Protein_Kinase_DOM (957-1221aa). Ron-V3 protein contains one Sema domain (31-522aa), one Protein_Kinase_ATP (954-980aa), one Protein_Kinase_TYR (1070-1082aa), and one Protein_Kinase_DOM (948-1212aa).

In the present invention, a search of ESTs deposited in dbEST at NCBI was performed to determine the presence of Ron gene variants in silico. The result of in silico analysis showed that one EST (GenBank accession number BG823879) was found to confirm the absence of 255bp region on Ron-V1, Ron-V2, and Ron-V3 nucleotide sequences; one EST (GenBank accession number AW009348) was found to confirm the absence of 137bp region on Ron-V1, Ron-V2, and Ron-V3 nucleotide sequences. Therefore, any Ron nucleotide fragments containing the immediately upstream and downstream sequences of the deleted 255bp region (2837-2838bp of Ron-V1; 2717-2718bp of Ron-V2; 2691-2692bp of Ron-V3) and/or 137bp region (3583-3584bp of Ron-V1; 3463-3464bp of Ron-V2; 3436-3437bp of Ron-V3) and/or 149bp region (3725-3726bp of Ron-V1; 3605-3606bp of Ron-V2; 3578-3579bp of Ron-V3) are the "gene targets" which may be used as probes to determine the presence of Ron-V1, Ron-V2, and Ron-V3 under high stringency conditions. An alternative approach is that any set of primers for amplifying the fragment containing the immediately upstream and downstream sequences of the deleted 255bp region (2837-2838bp of Ron-V1; 2717-2718bp of Ron-V2; 2691-2692bp of Ron-V3) and/or 137bp region (3583-3584bp of Ron-V1; 3463-3464bp of Ron-V2; 3436-3437bp of Ron-V3) and/or 149bp region (3725-3726bp of Ron-V1; 3605-3606bp of Ron-V2; 3578-3579bp of Ron-V3) may be used for determining the presence of the variants (Ron-V1, Ron-V2, and Ron-V3). On the other hand, any Ron nucleotide fragments containing the immediately upstream and downstream sequences of the deleted 120bp region (2678-2679bp of Ron-V2) and 147bp region (2678-2679bp of Ron-V3) are the "gene targets" which may be used as probes for determining the presence of Ron-V2 and Ron-V3, respectively, under high stringency conditions. An alternative approach is that any set of primers for amplifying the fragment containing the immediately upstream and downstream sequences of the deleted 120bp region (2678-2679bp of Ron-V2) and 147bp region (2678-2679bp of Ron-V3) may be used for determining the presence of Ron-V2 and Ron-V3, respectively.

According to the present invention, the polypeptides of the human Ron-V1, Ron-V2, and Ron-V3 and its fragments thereof may be produced through genetic engineering techniques. In this case, they are produced by appropriate host cells which have been transformed by DNAs that encode for the polypeptides or fragments thereof. The nucleotide sequence encoding the polypeptide of the human Ron-V1, Ron-V2, and Ron-V3 or their fragments thereof are inserted into an appropriate expression vector, i.e., a vector which contains the necessary elements for the transcription and translation of the inserted coding sequence in a suitable host. The nucleic acid sequence is inserted into the vector in a manner that will be expressed under appropriate conditions (e.g., in proper orientation and correct reading frame and with appropriate expression sequences, including an RNA polymerase binding sequence and a ribosomal binding sequence).

Any method that is known to those skilled in the art may be used to construct expression vectors containing sequences encoding the polypeptides of the human Ron-V1, Ron-V2, and Ron-V3 and appropriate transcriptional/translational control elements. These methods may include in vitro recombinant DNA and synthetic techniques, and in vivo genetic recombinants.

A variety of expression vector/host systems may be utilized to express the polypeptide-coding sequence. These include, but are not limited to, microorganisms such as bacteria transformed with recombinant bacteriophage, plasmid, or cosmid DNA expression vector; yeast transformed with yeast expression vector; insect cell systems infected with virus (e.g., baculovirus); plant cell system transformed with viral expression vector (e.g., cauliflower mosaic virus, CaMV, or tobacco mosaic virus, TMV); or animal cell system infected with virus (e.g., vaccina virus, adenovirus, etc.). Preferably, the host cell is a bacterium, and most preferably, the bacterium is E. coli.

Alternatively, the Polypeptides of the human Ron-V1, Ron-V2, and Ron-V3 or fragments thereof may be synthesized using chemical methods. For example, peptide synthesis can be performed using various solid-phase techniques. Automated synthesis may be achieved using the ABI 431A peptide synthesizer (Perkin-Elmer).

According to the present invention, the fragments of the polypeptides and nucleic acid sequences of the human Ron-V1, Ron-V2, and Ron-V3 are used as immunogens, primers or probes. Preferable, the purified fragments of the human Ron-V1, Ron-V2, and Ron-V3 are used. The fragments may be produced by enzyme digestion, chemical cleavage of isolated or purified polypeptide or nucleic acid sequences, or chemical synthesis. Thereafter, the fragments may be isolated or purified. Such isolated or purified fragments of the polypeptides and nucleic acid sequences can be used as immunogens, primers or probes.

The present invention further provides the antibodies which specifically bind one or more out-surface epitopes of the polypeptides of the human Ron-V1, Ron-V2, and Ron-V3.

According to the present invention, immunization of mammals with immunogens described herein, preferably humans, rabbits, rats, mice, sheep, goats, cows, or horses, is performed following procedures well known to those skilled in the art, for the purpose of obtaining antisera containing polyclonal antibodies or hybridoma lines secreting monoclonal antibodies.

Monoclonal antibodies can be prepared by standard techniques. Such techniques are disclosed, for example, in U.S. patent number 4,271,145 and U.S. patent number 4,196,265. An animal is immunized with the immunogen. Hybridomas are prepared by fusing spleen cells from the immunized animal with myeloma cells. The fusion products are screened for those producing antibodies that specifically bind to the immunogen. The positive hybridoma clones are isolated, and the monoclonal antibodies are recovered from those clones.

Immunization regimens for production of both polyclonal and monoclonal antibodies are well-known in the art. The immunogen may be injected by any of a number of routes, including subcutaneous, intravenous, intraperitoneal, intradermal, intramuscular, mucosal, or a combination thereof. The immunogen may be injected in soluble form, aggregate form, attached to a physical carrier, or mixed with an adjuvant, using methods and materials well-known in the art. The antisera and antibodies may be purified using well-known column chromatography methods.

According to the present invention, antibody fragments which contain specific binding sites for the polypeptides or fragments thereof may also be generated. For example, such fragments include, but are not limited to, F(ab')2 fragments produced by pepsin digestion of the antibody molecule and Fab fragments generated by reducing the disulfide bridges of the F(ab')2 fragments.

The subject invention also provides methods for diagnosing the diseases associated with Ron-V1, Ron-V2, or Ron-V3, particularly breast carcinoma, breast adenocarcinoma, cervix epidermoid carcinoma, cervix epitheloid carcinoma, colon adenocarcinoma, urinary bladder carcinoma, prostate carcinoma, esophagus epidermoid carcinoma and esophagus carcinoma, by utilizing the nucleic acid sequences, the polypeptide of the human Ron-V1, Ron-V2, or Ron-V3, or fragments thereof, and the antibodies against the polypeptides.

Since Ron-V1, Ron-V2, or Ron-V3 clones were isolated from a SW620 cDNA library, it is advisable that Ron-V1, Ron-V2, or Ron-V3 serve as a marker for the diagnosis of human colon adenocarcinoma. Thus, the expression levels/patterns of Ron-V1, Ron-V2, or Ron-V3 may be a useful indicator for screening patients suspected of having cancers or more specifically, breast carcinoma, breast adenocarcinoma, cervix epidermoid carcinoma, cervix epitheloid carcinoma, colon adenocarcinoma, urinary bladder carcinoma, prostate carcinoma, esophagus epidermoid carcinoma and esophagus carcinoma. This suggests that relative expression levels/patterns (mRNA or protein) may confer an increased susceptibility to cancers, and more preferably, to breast carcinoma, breast adenocarcinoma, cervix epidermoid carcinoma, cervix epitheloid carcinoma, colon adenocarcinoma, urinary bladder carcinoma, prostate carcinoma, esophagus epidermoid carcinoma and esophagus carcinoma. Fragments of Ron-V1, Ron-V2, or Ron-V3 transcripts (mRNAs) may be detected by RT-PCR approach. Polypeptides of Ron-V1, Ron-V2, or Ron-V3 may be determined by the binding of antibodies to these polypeptides. These approaches may be performed in accordance with conventional methods well known to persons skilled in the art. On the other hand, a method (RNAi; RNA interference) that is known to those skilled in the art may be used to interfere and degrade the targeted RNA using chemically synthesized double stranded short nucleic acid (about 23 nucleotides dsRNA) molecules (Montgomery et al. (1998) Proc Natl Acad Sci U S A. 95:15502-7). According to the present invention, the double stranded short nucleic acid molecules containing the sequences of the "gene targets": nucleotides 2837-2838, nucleotides 3583-3584, or nucleotides 3725-3726 of Ron-V1; nucleotides 2717-2718, nucleotides 3463-3464, or nucleotides 3605-3606 of Ron-V2; nucleotides 2691-2692, nucleotides 3436-3437, or nucleotides 3578-3579 of Ron-V3, which may be used to specifically interfere and degrade Ron-V1, Ron-V2, and Ron-V3 mRNAs. On the other hand, the double stranded short nucleic acid molecules containing the sequence of the "gene targets": nucleotides 2678-2679 of Ron-V2 or Ron-V3, may be used to specifically interfere and degrade Ron-V2 and Ron-V3 mRNA, respectively. After RNAi approach was conducted, the decreased transcripts or polypeptides may be used to interpret the presence of Ron-V1, Ron-V2, and Ron-V3 mRNAs.

According to the present invention, the expression of these gene variant mRNAs in sample may be determined by, but not limited to, RT-PCR. Using TRIZOL reagents (Life Technology), total RNA may be isolated from patient samples. Tissue samples (e.g., biopsy samples) are powdered under liquid nitrogen before homogenization. RNA purity and integrity are assessed by absorbance at 260/280 nm and by agarose gel electrophoresis. A set of primers can be designed to amplify the expected size of specific PCR fragments (gene targets) of Ron-V1, Ron-V2, and Ron-V3. For example, one of the primers is a fragment of the sequence (A fragment; forward primer) containing the gene targets: nucleotides 2837-2838, nucleotides 3583-3584, or nucleotides 3725-3726 of Ron-V1; nucleotides 2717-2718, nucleotides 3463-3464, or nucleotides 3605-3606 of Ron-V2; nucleotides 2691-2692, nucleotides 3436-3437, or nucleotides 3578-3579 of Ron-V3, and the other is a fragment (B fragment; reverse primer) designed from a reverse complementary strand of Ron-V1, Ron-V2, and Ron-V3 sequences at any location downstream of "A fragment". If "B fragment" is designed from a reverse complementary strand of the sequence containing the gene targets: nucleotides 2837-2838, nucleotides 3583-3584, or nucleotides 3725-3726 of Ron-V1; nucleotides 2717-2718, nucleotides 3463-3464, or nucleotides 3605-3606 of Ron-V2; nucleotides 2691-2692, nucleotides 3436-3437, or nucleotides 3578-3579 of Ron-V3, the "A fragment" is a fragment of the sequence at any location upstream of "B fragment". Alternatively, one primer (A fragment; forward primer) is at any location upstream of the sequence containing "gene targets" and the other is designed from a reverse complementary strand at any location downstream of the sequence containing "gene targets". PCR fragments are analyzed on a 1 % agarose gel using five microliters (10%) of the amplified products. The intensity of the signals may be determined by using the Molecular Analyst program (version 1.4.1; Bio-Rad). Thus, the relative expression level for each amplified PCR product may be calculated based on the intensity of signals.

The RT-PCR experiment may be performed according to the manufacturer instructions (Boehringer Mannheim). A 50µl reaction mixture containing 2µl total RNA (0.1 µg/µl), 1µl each primer (20 pM), 1µl each dNTP (10 mM), 2.5 µl DTT solution (100 mM), 10 µl 5X RT-PCR buffer, 1µl enzyme mixture, and 28.5 µl sterile distilled water may be subjected to the conditions such as reverse transcription at 60oC for 30 minutes followed by 35 cycles of denaturation at 94oC for 2 minutes, annealing at 60oC for 2 minutes, and extension at 68oC for 2 minutes. The RT-PCR analysis may be repeated twice to ensure reproducibility, for a total of three independent experiments.

The expression of gene variants can also be analyzed using Northern Blot hybridization approach. Specific fragments containing the sequences of the "gene targets" of the Ron-V1, Ron-V2, and Ron-V3 may be amplified by polymerase chain reaction (PCR) using primer set designed for RT-PCR. The amplified PCR fragment may be labeled and served as a probe to hybridize the membranes containing total RNAs extracted from the samples under the conditions of 55°C in a suitable hybridization solution for 3 hours. Blots may be washed twice in 2 x SSC, 0.1% SDS at room temperature for 15 minutes each, followed by two washes in 0.1 x SSC and 0.1 % SDS at 65°C for 20 minutes each. After these washes, blot may be rinsed briefly in suitable washing buffer and incubated in blocking solution for 30 minutes, and then incubated in suitable antibody solution for 30 minutes. Blots may be washed in washing buffer for 30 minutes and equilibrated in suitable detection buffer before detecting the signals. Alternatively, the presence of the "gene targets" of the gene variants (cDNAs or PCR) can be detected using microarray approach. The cDNAs or PCR products corresponding to the nucleotide sequences of the present invention may be immobilized on a suitable substrate such as a glass slide. Hybridization can be preformed using the labeled mRNAs extracted from samples. After hybridization, nonhybridized mRNAs are removed. The relative abundance of each labeled transcript, hybridizing to a cDNA/PCR product immobilized on the microarray, can be determined by analyzing the scanned images.

According to the present invention, the presence of the polypeptide of Ron-V1, Ron-V2, and Ron-V3 in samples may be determined by, but not limited to, the immunoassay which uses the antibody that specifically binds to the polypeptide. The polypeptides of the gene variants may be expressed in prokaryotic cells by using suitable prokaryotic expression vectors. The cDNA fragments of Ron-V1, Ron-V2, and Ron-V3 genes encoding the amino acid sequences may be PCR amplified using primer set designed with restriction enzyme digestion sites incorporated in the 5' and 3' ends. The PCR products can then be enzyme digested, purified, and inserted into the corresponding sites of prokaryotic expression vector in-frame to generate recombinant plasmids. Sequence fidelity of this recombinant DNA can be verified by sequencing. The prokaryotic recombinant plasmids may be transformed into host cells (e.g., E. coli BL21 (DE3)). Recombinant protein synthesis may be stimulated by the addition of 0.4 mM isopropylthiogalactoside (IPTG) for 3h. The bacterially-expressed proteins may be purified.

The polypeptide of the gene variant may be expressed in animal cells by using eukaryotic expression vectors. Cells may be maintained in Dulbecco's modified Eagle's medium (DMEM) supplemented with 10% fetal bovine serum (FBS; Gibco BRL) at 37oC in a humidified 5% CO2 atmosphere. Before transfection, the nucleotide sequence of each of the gene variant may be amplified with PCR primers containing restriction enzyme digestion sites and ligated into the corresponding sites of eukaryotic expression vector in-frame. Sequence fidelity of this recombinant DNA can be verified by sequencing. The cells may be plated in 12-well plates one day before transfection at a density of 5 x 104 cells per well. Transfections may be carried out using Lipofectaminutese Plus transfection reagent according to the manufacturer's instructions (Gibco BRL). Three hours following transfection, medium containing the complexes may be replaced with fresh medium. Forty-eight hours after incubation, the cells may be scraped into lysis buffer (0.1 M Tris HCl, pH 8.0, 0.1% Triton X-100) for purification of expressed proteins. After these proteins are purified, monoclonal antibodies against these purified proteins (Ron-V1, Ron-V2, and Ron-V3) may be generated using hybridoma technique according to the conventional methods (de StGroth and Scheidegger, (1980) J Immunol Methods 35:1-21; Cote et al. (1983) Proc Natl Acad Sci U S A 80: 2026-30; and Kozbor et al. (1985) J Immunol Methods 81:31-42).

According to the present invention, the presence of the polypeptides of Ron-V1, Ron-V2, and Ron-V3 in samples of the breast carcinoma, breast adenocarcinoma, cervix epidermoid carcinoma, cervix epitheloid carcinoma, colon adenocarcinoma, urinary bladder carcinoma, prostate carcinoma, esophagus epidermoid carcinoma and esophagus carcinoma may be determined by a Western blot analysis. Proteins extracted from samples may be separated by SDS-PAGE and transferred to suitable membranes such as polyvinylidene difluoride (PVDF) in transfer buffer (25 mM Tris-HCl, pH 8.3, 192 mM glycine, 20% methanol) with a Trans-Blot apparatus for 1h at 100 V (e.g., Bio-Rad). The proteins can be immunoblotted with specific antibodies. For example, membrane blotted with extracted proteins may be blocked with suitable buffers such as 3% solution of BSA or 3% solution of nonfat milk powder in TBST buffer (10 mM Tris-HCl, pH 8.0, 150 mM NaCl, 0.1% Tween 20) and incubated with monoclonal antibody directed against the polypeptides of gene variants. Unbound antibody is removed by washing with TBST for 5 X 1 minutes. Bound antibody may be detected using commercial ECL Western blotting detecting reagents.

The following examples are provided for illustration, but not for limiting the invention.

### EXAMPLES

### ANALYSIS OF HUMAN COLON ADENOCARCINOMA EST DATABASE

Expressed sequence tags (ESTs) generated from the large-scale PCR-based sequencing of the 5'-end of human colon adenocarcinoma cDNA clones were compiled and served as EST databases. Sequence comparisons against the nonredundant nucleotide and protein databases were performed using BLASTN and BLASTX programs, at the National Center for Biotechnology Information (NCBI) with a significance cutoff of p<10-10. ESTs representing putative Ron-V1, Ron-V2, and Ron-V3 genes were identified during the course of EST generation.

### ISOLATION OF CDNA CLONES

Three cDNA clones exhibiting EST sequences similar to the Ron gene were isolated from the human colon adenocarcinoma cDNA library and named Ron-V1, Ron-V2, and Ron-V3. The inserts of these clones were subsequently excised in vivo from the λZAP Express vector using the ExAssist/XLOLR helper phage system (Stratagene). Phagemid particles were excised by coinfecting XLI-BLUE MRF' cells with ExAssist helper phage. The excised pBluescript phagemids were used to infect E. coli XLOLR cells, which lack the amber suppressor necessary for ExAssist phage replication. Infected XLOLR cells were selected using kanamycin resistance. Resultant colonies contained the double stranded phagemid vector with the cloned cDNA insert. A single colony was grown overnight in LB-kanamycin, and DNA was purified using a Qiagen plasmid purification kit.

### FULL LENGTH NUCLEOTIDE SEQUENCING AND DATABASE COMPARISONS

Phagemid DNA was sequenced using the Taq dye-deoxy terminator cycle sequencing kit for the Applied Biosystems 377 sequencing system (PerkinElmer Life Sciences). Using the primer-walking approach, full-length sequence was determined. Nucleotide and protein searches were performed using BLAST against the non-redundant database of NCBI.

### IN SILICO SEQUENCE SIMILARITY ANALYSIS

The coding sequence for each cDNA clones was searched against the dbEST sequence database using the BLAST algorithm at the NCBI website. ESTs derived from dbEST sequence database were used as a source of information for transcript sequence similarity analysis. The accession numbers of the ESTs matching to the deleted sequence of Ron-V1, Ron-V2, and Ron-V3 were identified.

### RT-PCR EXPRESSION PATTERN ANALYSIS

The expression patterns of Ron-V1, Ron-V2, and Ron-V3 were conducted in human cell lines using RT-PCR. The human cell lines were WI38 (Lung, fetus); A549 (Lung adenocarcinoma); H661 (Large cell carcinoma, lung); H520 (Squamous cell carcinoma, lung); H209 (Small cell carcinoma, lung); JHH-4 (Hepatoma); SUP-T1 (T-cell lymophoblastic lymphoma); Daudi (Burkitt's lymophoma); Ramos (Burkitt's lymophoma); RAJI (Burkitt's lymophoma); ZR75-1 (Breast carcinoma); MDA-MB-231 (Breast adenocarcinoma); Hs 578T (Breast carcinoma); G5T/VGH (Glioblastoma multiforme); TSGH9201 (Gastric carcinoma); Ca Ski (Cervix epidermoid carcinoma); Hela S3 (Cervical epitheloid carcinoma); COLO 320 HSR (Colon adenocarcinoma); SW620 (Colon adenocarcinoma); TSGH8301 (Urinary bladder carcinoma); ES-2 (Ovarian carcinoma); DU145 (Brain prostate carcinoma); MIA paca-2 (Pancreatic carcinoma); CE48T/VGH (Esophagus epidermoid carcinoma); CE81T/VGH (Esophagus carcinoma well differentiated aquamous); HL-CZ (Promonocytic leukemia); Hs 181.tes (Normal testis). The purity and integrity of total RNA extracted from each of the cell lines were assessed by absorbance at 260/280 nm and by agarose gel electrophoresis. The forward and reverse primers for Ron gene variants were: 5'-GACTGAGTGTCTGCTAGCACGG-3' and 5'-ATGGCAGGGAGTGCATCTACGC-3'. The expected size of Ron-V1, Ron-V2, and Ron-V3 PCR fragments were 660bp, 540bp and 513bp.

Glyceraldehyde-3-phosphate dehydrogenase (GAPDH; accession No. M33197) was used for internal control. The forward and reverse primers for GAPDH were: 5'-TGGGTGTGAACCATGAGAAG-3' and 5'-GTGTCGCTGTTGAAGTCAGA-3'. The expected size of GAPDH PCR fragment was 472 bp. Briefly, a 50 µl reaction mixture containing 2µl total RNA (0.1 µg/µl), 1µl each primer (20 pM), 1µl each dNTP (10 mM), 2.5 µl DTT solution (100 mM), 10 µl 5X RT-PCR buffer, 1µl enzyme mixture and 28.5 µl sterile redistilled water were subjected to reverse transcription at 60°C for 30 min followed by 35 cycles of denaturation at 94°C for 2 min, annealing at 60°C for 2 min, and extension at 68°C for 2 min. Five microliters (10%) of the amplified products mixed with 1 µl of loading buffer were separated on a 1% horizontal agarose gel stained with ethidium bromide in 0.5X TAE buffer. The gel was electrophoresed at 100 V for 45 min.

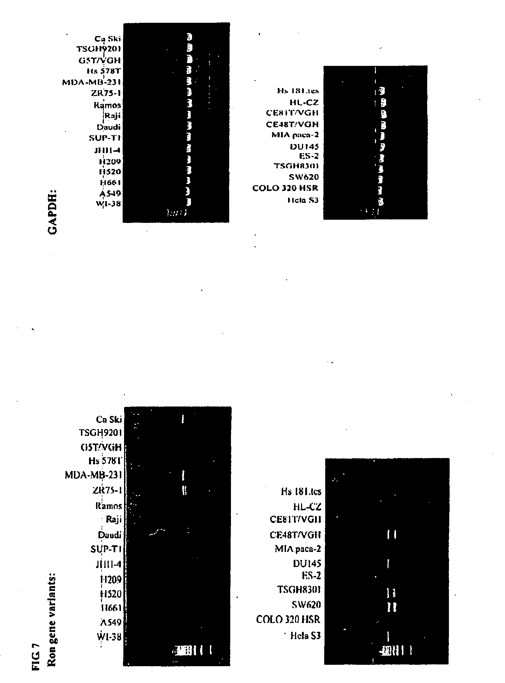

Figure 7 showed that Ron-V1 (660bp), Ron-V2 (540bp), and Ron-V3 (513bp) mRNAs were RT-PCR amplified using the primers described above. Shown on the left are 100 bp DNA ladder markers. The PCR fragments of Ron-V1 (660bp), Ron-V2 (540bp), and Ron-V3 (513bp) were confirmed by sequencing. Ron-V1 mRNA was expressed in the cell lines of cervix epitheloid carcinoma, colon adenocarcinoma, urinary bladder carcinoma, prostate carcinoma, esophagus carcinoma, and esophagus epidermoid carcinoma; Ron-V2 mRNA was expressed in the cell lines of breast carcinoma, breast adenocarcinoma, cervix epidermoid carcinoma, colon adenocarcinoma, and urinary bladder carcinoma; Ron-V3 mRNA was expressed in the cell lines of breast carcinoma, colon adenocarcinoma, urinary bladder carcinoma, esophagus carcinoma, and esophagus epidermoid carcinoma. The differential expression pattern among Ron-V1, Ron-V2, and Ron-V3 implied that they may be functionally different and may be a suitable marker for diagnosing human breast carcinoma, breast adenocarcinoma, cervix epidermoid carcinoma, cervix epitheloid carcinoma, colon adenocarcinoma, urinary bladder carcinoma, prostate carcinoma, esophagus epidermoid carcinoma and esophagus carcinoma.

### REFERENCES

All references are listed herein for the convenience of the reader.
1. Angeloni et al., The soluble sema domain of the RON receptor inhibits macrophage-stimulating protein-induced receptor activation. J. Biol. Chem. 279:3726-32, (2004).
2. Camp et al., RON, a tyrosine kinase receptor involved in tumor progression and metastasis.Ann Surg Oncol. 12:273-81, (2005).
3. Peace et al., Ron receptor signaling augments mammary tumor formation and metastasis in a murine model of breast cancer. Cancer Res. 65:1285-93, (2005).
4. Wang et al., Oncogenic and invasive potentials of human macrophage-stimulating protein receptor, the RON receptor tyrosine kinase. Carcinogenesis 24:1291-300, (2003).
5. Willett et al., Differential screening of a human chromosome 3 library identifies hepatocyte growth factor-like/macrophage-stimulating protein and its receptor in injured lung. Possible implications for neuroendocrine cell survival. J Clin Invest. 99:2979-91, (1997).

## Claims

1. An isolated polypeptide comprising the amino acid sequence of SEQ ID NO: 4.

2. An isolated polypeptide comprising the amino acid sequence of SEQ ID NO: 6.

3. An isolated polypeptide comprising the amino acid sequence of SEQ ID NO: 8.

4. An isolated nucleic acid encoding said polypeptide of Claim 1.

5. An isolated nucleic acid encoding said polypeptide of Claim 2.

6. An isolated nucleic acid encoding said polypeptide of Claim 3.

7. The isolated nucleic acid of Claim 4, comprising the nucleotide sequence of SEQ ID NO: 3.

8. The isolated nucleic acid of Claim 5, comprising the nucleotide sequence of SEQ ID NO: 5.

9. The isolated nucleic acid of Claim 6, comprising the nucleotide sequence of SEQ ID NO: 7.

10. An isolated Ron nucleotide fragment comprising at least 30 consecutive nucleotides selected from SEQ ID NO: 3, SEQ ID NO: 5, or SEQ ID NO: 7 and that includes a dinucleotide selected from the group consisting of nucleotides 2837-2838 of SEQ ID NO: 3, nucleotides 3583-3584 of SEQ ID NO: 3, and nucleotides 3725-3726 of SEQ ID NO: 3; nucleotides 2678-2679 of SEQ ID NO: 5, nucleotides 2717-2718 of SEQ ID NO: 5, nucleotides 3463-3464 of SEQ ID NO: 5, nucleotides 3605-3606 of SEQ ID NO: 5, nucleotides 2678-2679 of SEQ ID NO: 7, nucleotides 2691-2692 of SEQ ID NO: 7, nucleotides 3436-3437 of SEQ ID NO: 7, and nucleotides 3578-3579 of SEQ ID NO: 7.

11. An isolated polypeptide comprising the polypeptide encoded by the nucleic acid of claim 10.

12. An purified polypeptide comprising the polypeptide encoded by the nucleic acid of claim 10.

13. An expression vector comprising the nucleic acid of any one of claims 4 to 10.

14. A host cell comprising said expression vector of Claim 13.

15. A method for producing a RON variant polypeptide, comprising the steps of:
a) culturing said host cell of Claim 14 under a condition suitable for the expression of a RON variant polypeptide; and
b) recovering said RON variant polypeptide from the host cell culture.

16. An antibody that specifically binds to a polypeptide of any of the claims 11 to 12.

17. The antibody of Claim 16, which is a polyclonal or monoclonal antibody.

18. A method for detecting the presence of a RON nucleic acid selected from SEQ ID NO: 3, SEQ ID NO: 5, or SEQ ID NO: 7 in a mammal, which comprises the steps of:
a) amplifying RNA from a sample obtained from said mammal by reverse transcriptase-polymerase chain reaction (RT-PCR) to obtain a cDNA sample;
b) hybridizing said cDNA sample with the nucleic acid of claim 10; and
c) detecting said hybridization.

19. The method of Claim 18, wherein said hybridizing process is conducted by Northern blot or microarray to diagnose a plurality of cancers.

20. The method of Claim 19, wherein said plurality of cancers include breast carcinoma, breast adenocarcinoma, cervix epidermoid carcinoma, cervix epitheloid carcinoma, colon adenocarcinoma, urinary bladder carcinoma, prostate carcinoma, esophagus epidermoid carcinoma and esophagus carcinoma.

21. A method for detecting the presence of a RON polypeptide or fragment thereof in a mammal, which comprises the steps of:
a) contacting proteins from said mammal with an antibody that binds to a RON antigen comprising a polypeptide having at least 17 contiguous and unique amino acids from SEQ ID NO: 4, 6, or 8 that are not found in SEQ ID NO: 2; and
b) detecting antibody-RON antigen binding.

22. The method of Claim 21, wherein said antibody-antigen binding samples are detected by Western blot approach to help diagnose a plurality of cancers.

23. The method of Claim 22, wherein said plurality of cancers include breast carcinoma, breast adenocarcinoma, cervix epidermoid carcinoma, cervix epitheloid carcinoma, colon adenocarcinoma, urinary bladder carcinoma, prostate carcinoma, esophagus epidermoid carcinoma and esophagus carcinoma.

## Patentansprüche

1. Isoliertes Polypeptid, das die Aminosäuresequenz SEQ ID Nr. 4 aufweist.

2. Isoliertes Polypeptid, das die Aminosäuresequenz SEQ ID Nr. 6 aufweist.

3. Isoliertes Polypeptid, das die Aminosäuresequenz SEQ ID Nr. 8 aufweist.

4. Isolierte Nukleinsäure, die das Polypeptid nach Anspruch 1 codiert.

5. Isolierte Nukleinsäure, die das Polypeptid nach Anspruch 2 codiert.

6. Isolierte Nukleinsäure, die das Polypeptid nach Anspruch 3 codiert.

7. Isolierte Nukleinsäure nach Anspruch 4, die die Nukleotidsequenz SEQ ID Nr. 3 aufweist.

8. Isolierte Nukleinsäure nach Anspruch 5, die die Nukleotidsequenz SEQ ID Nr. 5 aufweist.

9. Isolierte Nukleinsäure nach Anspruch 6, die die Nukleotidsequenz SEQ ID Nr. 7 aufweist.

10. Isoliertes RON-Nukleotidfragment, das mindestens 30 aufeinanderfolgende Nukleotide aufweist, die aus SEQ ID Nr. 3, SEQ ID Nr. 5 oder SEQ ID Nr. 7 ausgewählt sind, und das ein Dinukleotid aufweist, das aus der Gruppe ausgewählt ist, die aus Nukleotiden 2837-2838 der SEQ ID Nr. 3, Nukleotiden 3583-3584 der SEQ ID Nr. 3 und Nukleotiden 3725-3726 der SEQ ID Nr. 3; Nukleotiden 2678-2679 der SEQ ID Nr. 5, Nukleotiden 2717-2718 der SEQ ID Nr. 5, Nukleotiden 3463-3464 der SEQ ID Nr. 5, Nukleotiden 3605-3606 der SEQ ID Nr. 5, Nukleotiden 2678-2679 der SEQ ID Nr. 7, Nukleotiden 2691-2692 der SEQ ID Nr. 7, Nukleotiden 3436-3437 der SEQ ID Nr. 7, und Nukleotiden 3578-3579 der SEQ ID Nr. 7 besteht.

11. Isoliertes Polypeptid, welches das Polypeptid aufweist, das durch die Nukleinsäure nach Anspruch 10 codiert wird.

12. Gereinigtes Polypeptid, welches das Polypeptid aufweist, das durch die Nukleinsäure nach Anspruch 10 codiert wird.

13. Expressionsvektor, der die Nukleinsäure nach einem der Ansprüche 4 bis 10 aufweist.

14. Wirtszelle, die den Expressionsvektor nach Anspruch 13 aufweist.

15. Verfahren zur Herstellung eines RON-Varianten-Polypeptids, folgende Schritte umfassend:
a) Züchtung der Wirtszelle nach Anspruch 14 unter Bedingungen, die für die Expression eines RON-Varianten-Polypeptids geeignet sind; und
b) Gewinnung des RON-Varianten-Polypeptids aus der Wirtszellenkultur.

16. Antikörper, der sich spezifisch an ein Polypeptid nach einem der Ansprüche 11 bis 12 bindet.

17. Antikörper nach Anspruch 16, bei dem es sich um einen polyklonalen oder monoklonalen Antikörper handelt.

18. Verfahren zur Erfassung des Vorhandenseins einer aus SEQ ID Nr. 3, SEQ ID Nr. 5 oder SEQ ID Nr. 7 ausgewählten RON-Nukleinsäure in einem Säugetier, das die folgenden Schritte umfasst:
a) Amplifizierung von RNA aus einer dem Säugetier entnommenen Probe durch eine Reverse Transkriptase-Polymerase-Kettenreaktion (RT-PCR), um eine cDNA-Probe zu erhalten;
b) Hybridisierung der cDNA-Probe mit der Nukleinsäure nach Anspruch 10; und
c) Detektieren der Hybridisierung.

19. Verfahren nach Anspruch 18, wobei der Hybridisierungsprozess mittels Northern Blot oder eines Mikroarrays ausgeführt wird, um mehrere Krebsarten zu diagnostizieren.

20. Verfahren nach Anspruch 19, wobei die mehreren Krebsarten Mammakarzinom, Brustadenokarzinom, Gebärmutterhals-Epidermoidkarzinom, Gebärmutterhals-Epitheloidkarzinom, Dickdarmadenokarzinom, Harnblasenkarzinom, Prostatakarzinom, Speiseröhren-Epidermoidkarzinom und Speiseröhrenkarzinom umfassen.

21. Verfahren zur Erfassung des Vorhandenseins eines RON-Polypeptids oder Fragments hiervon in einem Säugetier, welches die folgenden Schritte umfasst:
a) Inkontaktbringen der von dem Säugetier stammenden Proteine mit einem Antikörper, der sich an ein RON-Antigen bindet, das ein Polypeptid umfasst, welches mindestens 17 zusammenhängende und eindeutige Aminosäuren aus SEQ ID Nr. 4, 6 oder 8 umfasst, die sich nicht in SEQ ID Nr. 2 finden; und
b) Detektieren der Bindung Antikörper/RON-Antigen.

22. Verfahren nach Anspruch 21, wobei die Proben der Bindung Antikörper/Antigen durch die Western-Blot-Methode detektiert werden, um die Diagnose mehrerer Krebsarten zu unterstützen.

23. Verfahren nach Anspruch 22, wobei die mehreren Krebsarten Mammakarzinom, Brustadenokarzinom, Gebärmutterhals-Epidermoidkarzinom, Gebärmutterhals-Epitheloidkarzinom, Dickdarmadenokarzinom, Harnblasenkarzinom, Prostatakarzinom, Speiseröhren-Epidermoidkarzinom und Speiseröhrenkarzinom umfassen.

## Revendications

1. Un polypeptide isolé comprenant la séquence d'acides aminés de SEQ ID NO: 4.

2. Un polypeptide isolé comprenant la séquence d'acides aminés de SEQ ID NO: 6.

3. Un polypeptide isolé comprenant la séquence d'acides aminés de SEQ ID NO: 8.

4. Un acide nucléique isolé codant ledit polypeptide de la revendication 1.

5. Un acide nucléique isolé codant ledit polypeptide de la revendication 2.

6. Un acide nucléique isolé codant ledit polypeptide de la revendication 3.

7. L'acide nucléique isolé de la revendication 4, comprenant la séquence nucléotidique de SEQ ID NO: 3.

8. L'acide nucléique isolé de la revendication 5, comprenant la séquence nucléotidique de SEQ ID NO: 5.

9. L'acide nucléique isolé de la revendication 6, comprenant la séquence nucléotidique de SEQ ID NO: 7.

10. Un fragment nucléotidique Ron isolé comprenant au moins 30 nucléotides consécutifs sélectionnés parmi SEQ ID NO: 3, SEQ ID NO: 5, ou SEQ ID NO: 7 et incluant un dinucléotide sélectionné dans le groupe constitué de nucléotides 2837-2838 de SEQ ID NO: 3, nucléotides 3583-3584 de SEQ ID NO: 3, et nucléotides 3725-3726 de SEQ ID NO: 3 ; nucléotides 2678-2679 de SEQ ID NO: 5, nucléotides 2717-2718 de SEQ ID NO: 5, nucléotides 3463-3464 de SEQ ID NO: 5, nucléotides 3605-3606 de SEQ ID NO: 5, nucléotides 2678-2679 de SEQ ID NO: 7, nucléotides 2691-2692 de SEQ ID NO: 7, nucléotides 3436-3437 de SEQ ID NO: 7, et nucléotides 3578-3579 de SEQ ID NO: 7.

11. Un polypeptide isolé comprenant le polypeptide codé par l'acide nucléique de la revendication 10.

12. Un polypeptide purifié comprenant le polypeptide codé par l'acide nucléique de la revendication 10.

13. Un vecteur d'expression comprenant l'acide nucléique de l'une quelconque des revendications 4 à 10.

14. Une cellule hôte comprenant ledit vecteur d'expression de la revendication 13.

15. Procédé de production d'un polypeptide variant RON, comportant les étapes de :
a) mise en culture de ladite cellule hôte de la revendication 14 dans un état adapté à l'expression d'un polypeptide variant RON ; et
b) récupération dudit polypeptide variant RON à partir de la culture de cellule hôte.

16. Anticorps se liant spécifiquement à un polypeptide de l'une quelconque des revendications 11 à 12.

17. L'anticorps de la revendication 16, qui est un anticorps polyclonal ou monoclonal.

18. Procédé permettant de détecter la présence d'un acide nucléique RON sélectionné parmi SEQ ID NO: 3, SEQ ID NO: 5, ou SEQ ID NO: 7 chez un mammifère, comportant les étapes de :
a) amplification d'ARN à partir d'un échantillon obtenu sur ledit mammifère par réaction en chaîne par transcriptase-polymérase inverse (RT-PCR) pour obtenir un échantillon d'ADN-c ;
b) hybridation dudit échantillon d'ADN-c avec l'acide nucléique de la revendication 10 ; et
c) détection de ladite hybridation.

19. Le procédé de la revendication 18, où ledit processus d'hybridation est réalisé par northern blot ou micromatrice pour diagnostiquer une pluralité de cancers.

20. Le procédé de la revendication 19, où ladite pluralité de cancers inclut le carcinome du sein, l'adénocarcinome du sein, le carcinome épidermoïde du col de l'utérus, le carcinome épithéloïde du col de l'utérus, l'adénocarcinome du côlon, le carcinome de la vessie urinaire, le carcinome de la prostate, le carcinome épidermoïde de l'oesophage et le carcinome de l'oesophage.

21. Procédé permettant de détecter la présence d'un polypeptide RON ou fragment de celui-ci chez un mammifère, comportant les étapes de :
a) mise en contact de protéines issues dudit mammifère avec un anticorps qui se lie à un antigène RON comprenant un polypeptide présentant au moins 17 acides aminés contigus et uniques provenant de SEQ ID NO: 4, 6 ou 8 et ne se trouvant pas dans SEQ ID NO: 2 ; et
b) détection de liaisons anticorps-antigène RON.

22. Le procédé de la revendication 21, où lesdits échantillons de liaison anticorps-antigène sont détectés par approche Western blot pour aider à diagnostiquer une pluralité de cancers.

23. Le procédé de la revendication 22, où ladite pluralité de cancers inclut le carcinome du sein, l'adénocarcinome du sein, le carcinome épidermoïde du col de l'utérus, le carcinome épithéloïde du col de l'utérus, l'adénocarcinome du côlon, le carcinome de la vessie urinaire, le carcinome de la prostate, le carcinome épidermoïde de l'oesophage et le carcinome de l'oesophage.
